# FASCICULE DE BREVET EUROPEEN

(11) **EP 3 430 152 B1**
(45) Date de publication et mention de la délivrance du brevet: **02.02.2022**
(21) Numéro de dépôt: 17710305.8
(22) Date de dépôt: 15.03.2017
(51) Int. Cl.: C12P 5/02, C12M 1/107, C02F 11/04, C02F 11/10, C10B 53/02

(54) **DISPOSITIF DE TRANSFORMATION PAR VOIE BIOLOGIQUE DE GAZ DE PYROGAZÉIFICATION EN BIOGAZ**
VORRICHTUNG ZUR BIOLOGISCHE VERARBEITUNG VON PYROLYSE- VERGASUNG ZU BIOGAS
DEVICE FOR THE BIOLOGICAL TRANSFORMATION OF PYROGASIFICATION GAS INTO BIOGAS

(30) Priorité: 15.03.2016 FR 1652182
(43) Date de publication de la demande: 23.01.2019
(73) Titulaire: YANNCO, 92800 Puteaux (FR)
(72) Inventeur: MERCIER, Yann, 92800 Puteaux (FR)
(74) Mandataire: Casalonga
(86) Numéro de dépôt international: PCT/EP2017/056126
(87) Numéro de publication internationale: WO 2017/158024

(56) Documents cités:
- WO-A1-2015/003273
- US-A1- 2012 073 199
- US-A1- 2015 118 723
- HÜBNER TOBIAS ET AL: "Integration of pyrolysis and anaerobic digestion - Use of aqueous liquor from digestate pyrolysis for biogas production", BIORESOURCE TECHNOLOGY, vol. 183, 14 février 2015 (2015-02-14), pages 86-92, XP029203391, ISSN: 0960-8524, DOI: 10.1016/J.BIORTECH.2015.02.037
- PARRY D.L. ET AL.: "Pyrolysis of Dried Biosolids for Increased Biogas Production", PROCEEDINGS OF THE WATER ENVIRONMENT FEDERATION, RESIDUALS AND BIOSOLIDS, vol. 12, 2012, pages 1128-1139, XP008182469,
- F. MONLAU ET AL: "New opportunities for agricultural digestate valorization: current situation and perspectives", ENERGY & ENVIRONMENTAL SCIENCE, vol. 8, no. 9, 1 janvier 2015 (2015-01-01) , pages 2600-2621, XP055325365, UK ISSN: 1754-5692, DOI: 10.1039/C5EE01633A

## Description

La présente invention concerne un dispositif de transformation par voie biologique de gaz de pyrogazéification, issu d'un dispositif de pyrogazéification, en biogaz constitué principalement de méthane et de dioxyde de carbone.

L'invention concerne également un procédé de transformation par voie biologique de gaz de pyrogazéification, issu d'un procédé de pyrogazéification, en biogaz.

La pyrolyse consiste généralement à traiter thermiquement des matières organiques en absence d'oxygène pour produire majoritairement un mélange de gaz incondensables, des huiles et du charbon (char). Les proportions entre ces trois phases principales dépendent avant tout des conditions opératoires, notamment de la température, de la vitesse de chauffage des matières et du temps de séjour dans le réacteur de pyrolyse. Par exemple, à une faible température (généralement autour de 300 à 500°C), vitesse lente de chauffage des matières et long temps de séjour dans le réacteur, les teneurs en huile et en charbon sont favorisées. De manière alternative, à une température élevée (généralement autour de 500 à 900°C), vitesse rapide de chauffage et faible temps de séjour dans le réacteur, la phase gaz est majoritaire.

La gazéification consiste généralement à transformer en gaz les phases charbon et huile produites lors de l'étape de pyrolyse, par ajout d'une quantité faible de matière oxydante, généralement de l'air ou de la vapeur d'eau, ce qui a pour effet d'augmenter la température, parfois jusqu'à des températures supérieures à 1.200°C, et permet de récupérer sous forme de gaz la plus grande partie possible de l'énergie contenue dans ces deux phases. La pyrogazéification désigne généralement un procédé comprenant une étape de pyrolyse suivie d'une étape de gazéification.

Au sens de la présente invention, le terme de pyrogazéification désigne indistinctement un procédé de pyrolyse, de gazéification ou de pyro gazéification.

Le gaz de pyrogazéification, obtenu en sortie de l'unité de pyrogazéification, où il est généralement à une température élevée, entraîne le plus souvent avec lui des huiles mais également des goudrons, des gaz condensables de diverses natures ainsi que différents types de particules fines solides, en particulier des cendres, des particules de charbon et des particules minérales. Ces sous-produits sont présents dans le gaz de pyrogazéification en sortie du réacteur de pyrogazéification dans des proportions très variables selon les matières traitées et les conditions opératoires, en particulier en fonction de la température de la réaction.

Les matières susceptibles d'être valorisées dans les procédés de pyrogazéification sont de nature très diverses. Elles correspondent en général à des matières d'origine organique non fossiles, transformées ou non, ou bien à des matières organiques dérivées du pétrole. Parmi les grandes familles de matières d'origine organique non fossiles opportunément valorisables par pyrogazéification, on peut citer principalement et de manière non exhaustive les biomasses végétales d'origine agricole (le bois, les pailles, les menu-pailles et autres résidus de culture, les cultures énergétiques etc.), les déchets industriels banals non inertes et les déchets issus d'une collecte sélective de matières organiques d'origine municipale, (déchets de bois, papiers, cartons, textiles, etc.) ou encore les déchets d'activités de d'épuration (boues de stations d'épuration, etc.). Les matières dérivées du pétrole valorisables sont principalement des déchets d'objets en matière plastiques (résidus urbains d'objets en plastique de la vie courante, sac et autres emballages plastique, pneus, résidus de décharges automobiles etc.).

En fonction du type de matières organiques à valoriser, de leur qualité, de leur quantité, de la qualité du gaz de pyrogazéification que l'on souhaite obtenir, de son usage prévu, et en fonction des technologies privilégiées, les types de procédés de pyrogazéification mis en œuvre sont nombreux et peuvent être très différents les uns des autres. De même, les modes de valorisation des gaz de pyrogazéification sont nombreux et variés.

Par exemple, le gaz de pyrogazéification peut être brûlé dans une chaudière pour produire de l'eau chaude ou de la vapeur, qui peuvent être destinées à être mise en œuvre dans un procédé industriel, par exemple pour chauffer des utilités, ou encore dans une boucle de chaleur urbaine, pour chauffer des bâtiments.

En variante, le gaz de pyrogazéification peut aussi être brûlé dans une chaudière pour alimenter en vapeur une turbine produisant de l'énergie électrique.

Par ailleurs, le gaz de pyrogazéification peut également être transformé en un mélange de gaz incondensables relativement purs, appelé gaz de synthèse, dénommé communément syngaz, constitué principalement de monoxyde de carbone, de dihydrogène, communément appelé hydrogène, de méthane et de dioxyde de carbone, afin d'alimenter un moteur de cogénération en vu de produire principalement de l'énergie électrique et de façon secondaire de la chaleur. Pour ce faire, le gaz de pyrogazéification est au préalable refroidi et lavé pour obtenir du syngaz.

Cette dernière solution est intéressante car l'énergie électrique a généralement une plus grande valeur que l'énergie thermique et est plus facilement transportable. Les unités de production peuvent ainsi être installées dans des endroits où les besoins de chaleur sont limités.

Par « gaz de pyrogazéification », on entend donc au sens de la présente invention un mélange de gaz incondensables, obtenu suite à un procédé de pyrogazéification, entraînant et contenant des huiles, des goudrons, des gaz condensables et des particules fines solides, notamment des cendres, des particules de charbon et des particules minérales.

Par « gaz de synthèse ou syngaz », on entend au sens de la présente invention un mélange de gaz incondensables à température ambiante, constitué principalement de monoxyde de carbone, d'hydrogène, de méthane et de dioxyde de carbone. Le syngaz peut aussi contenir des proportions généralement faibles d'autres gaz incondensables, comme de l'azote gazeux, ou des hydrocarbures plus lourds que le méthane, de type CnHm. Le syngaz est obtenu après refroidissement et lavage du gaz de pyrogazéification. En d'autres termes, le gaz de synthèse ou syngaz correspond à un gaz de pyrogazéification qui a été préalablement refroidi et lavé afin de minimiser la teneur en sous-produits solides, liquides ou condensables.

Par « sous-produits du gaz de pyrogazéification >> ou SPGP, on entend au sens de la présente invention l'ensemble des huiles, des goudrons, des gaz condensés et des particules fines solides, séparées du syngaz et récupérés sous forme liquide ou solide après refroidissement et lavage du gaz de pyrogazéification. Ainsi, une fois récupérés, les sous-produits du gaz de pyrogazéification sont généralement des produits liquides contenant une certaine quantité de matières solides en suspension.

La nature des sous-produits contenus dans le gaz de pyrogazéification, en particulier les sous-produits gras, rend généralement difficiles les manipulations du gaz de pyrogazéification. Il en résulte que l'utilisation du gaz de pyrogazéification présente un certain nombre de difficultés.

Tout d'abord, le gaz de pyrogazéification est un gaz très encrassant, en raison de la présence des sous-produits qui le constituent, notamment les goudrons et les huiles qui ont souvent une viscosité élevée, lesquels peuvent provoquer :
- l'encrassement des tuyauteries et des équipements avant l'introduction du gaz de pyrogazéification dans les chaudières,
- l'encrassement des brûleurs de chaudière, ce qui peut poser des difficultés de réglage de la combustion,
- l'encrassement des éventuels équipements de lavage de gaz, notamment avant l'introduction du gaz dans des unités de cogénération, et
- l'encrassement des unités de cogénération.

Il résulte de ces difficultés que de nombreuses unités de pyrogazéification sont fréquemment arrêtées.

De plus, le gaz de pyrogazéification présente généralement l'inconvénient d'être corrosif, en particulier en raison de la présence fréquente de sulfure d'hydrogène (H₂S) ou d'acide chlorhydrique (HCl), pouvant conduire à la corrosion des équipements décrits précédemment. Les sous-produits du gaz de pyrogazéification ont généralement un pH acide, souvent compris entre 2 et 5.

Ainsi, pour être utilisé en tant que carburant dans des unités de cogénération, le gaz de pyrogazéification doit donc nécessairement être lavé et épuré de façon poussée afin d'obtenir un syngaz exempt de matières encrassantes et/ou corrosives, incompatibles avec le fonctionnement d'un moteur à explosion.

A cet effet, il existe des systèmes de lavage du gaz de pyrogazéification mis en œuvre par des procédés mécaniques, thermiques et/ou chimiques (filtres à manche, filtres à sable, filtres céramique, cyclones, lavage à l'huile, lavage à la chaux, lavage électrostatique, traitements thermiques, etc.). Toutefois, de tels systèmes sont généralement coûteux en termes d'investissement et d'exploitation, et sont souvent d'une fiabilité limitée, tant le caractère encrassant des gaz de pyrogazéification est élevé. Ainsi l'exploitation de certaines unités existantes de pyrogazéification est fréquemment suspendue en raison du fonctionnement insatisfaisant et du coût des systèmes de lavage mécaniques, thermiques et/ou chimiques.

En outre, des procédés de craquage des goudrons, présents dans les gaz de pyrogazéification, ont été développés pour résoudre ce type de problème, avec de bons résultats. Cependant de tels procédés sont, eux aussi, très coûteux en termes d'investissement et d'exploitation, notamment en raison du recours à des températures très élevées, souvent supérieures à 1.200°C. Pour cette raison, le recours à un système de refroidissement et/ou de lavage du gaz de pyrogazéification par injection de celui-ci dans des liquides de refroidissement et/ou de lavage, et/ou par aspersion de celui-ci dans lesdits liquides, s'avère être une solution souvent plus efficace, plus fiable et moins coûteuse en termes d'investissement et d'exploitation.

Toutefois, de tels systèmes de refroidissement et/ou de lavage en voie liquides posent aussi des problèmes car le mélange des sous-produits du gaz de pyrogazéification avec les liquides de refroidissement et/ou de lavage transforment ces liquides en déchets, lesquels sont difficiles à valoriser de manière rentable, et souvent coûteux à éliminer.

Ainsi la solution consistant à transformer le gaz de pyrogazéification en un syngaz utilisable dans une unité de cogénération génère des contraintes importantes en matière de coûts d'investissement, de coûts d'exploitation et de fiabilité.

La valorisation du gaz de pyrogazéification peut aussi poser des problèmes environnementaux, dans la mesure où celui-ci peut contenir des matières dont la combustion dans les chaudières ou les unités de cogénération peut produire des fumées toxiques, notamment en cas de pyrogazéification de déchets d'origine urbaine ou industrielle (cartons, plastiques, bois souillés, etc.). Dans ces conditions, des équipements de lavages de fumée sont nécessaires afin de réduire leur caractère toxique, lesquels équipements sont généralement très coûteux en investissement et en exploitation.

Enfin, le gaz de pyrogazéification peut être à l'origine de difficultés d'exploitation des équipements de valorisation, dans la mesure où le syngaz produit peut être de composition très variable selon les matières organiques traitées et le mode opératoire mis en œuvre. En particulier, les proportions de monoxyde de carbone, d'hydrogène, de méthane et de dioxyde de carbone sont difficiles à contrôler et peuvent varier sensiblement. Dans ces conditions, le réglage et la constance de combustion du syngaz dans les chaudières, et surtout dans les unités de cogénération, s'avèrent souvent problématiques.

Une valorisation encore plus intéressante du gaz de pyrogazéification peut être de transformer le syngaz en biogaz par le biais de procédés de méthanation.

Par procédé de « méthanation » on nomme tout procédé visant à transformer de l'hydrogène en biogaz par réaction avec du monoxyde de carbone et/ou du dioxyde de carbone. Les réactions mises en œuvre sont les suivantes :

CO + 3H₂ → CH₄ + H₂O

CO₂ + 4H₂ → CH₄ + 2H₂O

Au sens de la présente invention, par méthanation, on désigne les procédés ayant pour objet la transformation du syngaz en méthane, ou plus largement, en présence d'excès de dioxyde de carbone, les procédés ayant pour objet la transformation du syngaz en biogaz, lequel est un mélange essentiellement composé de méthane et de dioxyde de carbone. Le biogaz peut être lui-même transformé en biométhane suite à une opération de purification (ou « upgrading » en langue anglaise).

L'épuration du biogaz est un procédé de séparation du méthane et du dioxyde de carbone (et des autres gaz minoritaires) qui permet la production d'un biométhane assez pur pour le rendre apte à être injecté dans les réseaux de gaz naturel (généralement > 97%), ou à servir de carburant dans les transports routiers (généralement > 85%).

Il existe deux groupes de procédés de méthanation, les procédés de méthanation par transformation catalytique et les procédés de méthanation par voie biologique, ou biométhanation.

Les procédés de méthanation par transformation catalytique sont décrits dans la littérature scientifique. Ils ont fait l'objet de nombreuses expériences de laboratoire, et ont été mis en œuvre concrètement par la construction de quelques unités de tailles industrielles qui fonctionnent depuis peu de temps.

Cependant, la méthanation par transformation catalytique reste une technologie très coûteuse en investissements et en exploitation car elle repose sur des procédés faisant appel à des températures et des pressions élevées, et qui nécessitent l'utilisation de catalyseurs coûteux comme le nickel.

De plus, pour que la méthanation du syngaz par transformation catalytique fonctionne correctement, il est nécessaire de laver et d'épurer au préalable le gaz de pyrogazéification de façon poussée. Une telle opération engendre une nouvelle fois des contraintes de coûts d'investissement et de coûts d'exploitation pouvant remettre en cause la viabilité économique de ces procédés.

Les procédés de méthanation par voie biologique, dits procédés de biométhanation, sont également décrits dans la littérature scientifique et ont été validés par quelques expériences de laboratoire.

De tels procédés consistent à introduire le syngaz dans un réacteur de biométhanation afin de le transformer en biogaz.

Par ailleurs, il existe également d'autres procédés qui proposent d'introduire directement le gaz de pyrogazéification brut, c'est-à-dire sans traitement préliminaire préalable et contenant donc les sous-produits du gaz de pyrogazéification, dans une unité de méthanisation afin de transformer par voie biologique ledit gaz de pyrogazéification en biogaz.

Au sens de l'invention, une telle opération correspond à une transformation biologique du gaz de pyrogazéification en biogaz.

Par « méthanisation », on entend, au sens de la présente invention, tout procédé de transformation par voie biologique anaérobie de matières organiques biodégradables solides ou liquides en un biogaz essentiellement composé de méthane et de dioxyde de carbone.

Les procédés de méthanisation sont mis en œuvre par l'introduction des matières organiques à traiter dans des unités de méthanisation, généralement constituée d'un ou plusieurs digesteurs anaérobies. Ces digesteurs contiennent du digestat, constitué principalement d'eau, de matière organique non digérée, et d'un très grand nombre de bactéries anaérobies, appartenant à un très grand nombre de familles différentes, susceptibles de réaliser ensemble la fermentation anaérobie des matières organiques à transformer en biogaz. Les matières organiques à traiter sont mises en contact intime avec les bactéries présentes dans le digestat, généralement par agitation ou percolation du digestat dans les matières organiques, dans des conditions généralement contrôlées (température, concentrations etc.) généralement pendant plusieurs semaines.

Lorsque les matières organiques traitées ne contiennent pas de substances toxiques ou indésirables non biodégradables, le digestat excédentaire, produit par les unités de méthanisation, est un produit humide riche en matières organiques partiellement stabilisées et en micro-nutriments. Aussi, le digestat est souvent susceptible d'être utilisé ultérieurement comme amendement organique sur des terres agricoles.

La méthanisation peut s'appliquer notamment à tous les types de biomasses biodégradables ordinaires, tels que des produits ou les déchets issus de l'agriculture, des industries agroalimentaires ou encore les déchets alimentaires urbains ou de la restauration. La méthanisation peut aussi s'appliquer, sous certaines conditions plus strictes, à des déchets plus complexes, issus par exemple des industries chimiques, pétrochimiques, pharmaceutiques ou cosmétiques. La méthanisation peut, en outre, s'appliquer, sous certaines conditions, aux sous-produits des gaz de pyrogazéification de la plupart des matières organiques.

Cependant, il s'avère que l'introduction de gaz de pyrogazéification brut directement dans un réacteur de méthanisation pour le transformer par voie biologique en biogaz pose un certain nombre de problèmes. En effet, le gaz de pyrogazéification est très agressif pour les populations bactériennes, en raison de la température élevée du gaz en sortie de l'unité de pyrogazéification et surtout de la présence des sous-produits du gaz de pyrogazéification, lesquels peuvent être toxiques à certaines concentrations, et donc susceptibles d'inhiber les diverses réactions de transformation biologique du gaz de pyrogazéification en biogaz et de tuer certaines familles de bactéries responsables de ces réactions.

Par exemple, les sous-produits du gaz de la pyrolyse du bois, souvent appelés huiles de pyrolyse ou bio-huiles, sont constitués d'un mélange extrêmement complexe d'eau et de plus de deux cent composés oxygénés, parmi lesquels des fragments de lignine (lignine pyrolytique), des acides organiques (acétique, propionique, butyrique, formique, etc.), des aldéhydes et hydroxy-aldéhydes (formaldéhyde, acetaldéhyde, etc.), des cétones et hydroxy-cétones (acétone, cyclo-pentanone, etc.), des phénols (phénol, cresols, quaiacols, etc.), des alcools (méthanols, éthanol, etc.), des furanes (furfurals, etc.), etc. Or, il s'avère que la majorité de ces substances organiques sont toxiques pour certaines populations de bactéries au dessus de certaines concentrations. De plus, les proportions de chacune de ces substances dans les bio-huiles varient sensiblement en fonction du type de bois traité, de la température de traitement, et du type de procédé mis en œuvre (avec ou sans injection d'air, de vapeur etc.).

Dans cette circonstance, il est donc, ici encore, nécessaire de procéder à un refroidissement et un lavage préalable du gaz de pyrogazéification afin de séparer les sous-produits du gaz de pyrogazéification et d'obtenir un syngaz moins agressif pour la flore bactérienne de l'unité de biométhanation, ce qui engendre, une nouvelle fois, des contraintes en termes de coûts d'investissement et de coûts d'exploitation, et peut limiter la fiabilité de ces procédés.

En outre, le syngaz, même convenablement lavé et épuré, peut encore être agressif pour certaines familles de bactéries au dessus de certaines concentrations, ce qui peut entraîner des difficultés d'exploitation, en particulier concernant le dosage du syngaz dans l'unité de biométhanation. Ainsi la fiabilité des procédés de biométhanation reste faible et comporte des risques élevés d'inhibition ou de mortalité pour certaines populations bactériennes, notamment les plus fragiles.

De plus, le gaz de pyrogazéification peut contenir des matières organiques non biodégradables impropres à un usage agricole, notamment en cas de pyrogazéification de déchets d'origine urbaine (plastiques, bois souillés, etc.). Dans ces conditions, la totalité du ou des digestats, produite par le dispositif objet de l'invention, serait rendue inutilisable en usage agricole, ce qui générerait des coûts prohibitifs d'épuration, voire d'élimination de ces digestats.

La biométhanation du syngaz peut aussi être limitée par la difficulté de la dissolution de grandes quantités de monoxyde de carbone, d'hydrogène et de dioxyde de carbone dans un volume de liquide limité. En effet, le monoxyde de carbone, l'hydrogène et le dioxyde de carbone ont une faible capacité de dilution dans l'eau. Il peut arriver que les flores bactériennes de biométhanation soient efficaces, mais que leur action soit limitée par l'insuffisance du transfert de masses gaz-liquide, c'est à dire par une capacité trop faible de l'installation à transférer une quantité suffisante d'hydrogène, de monoxyde de carbone et/ou de dioxyde de carbone dans le milieu liquide, et que finalement les bactéries ne soient pas mises en contact avec les produits qu'elles doivent transformer. Dans ce cas, la biométhanation du syngaz peut être une réaction incomplète, la totalité du monoxyde de carbone et de l'hydrogène présents dans le syngaz n'étant pas transformée en méthane et en dioxyde de carbone.

Ainsi la publication de Marshall D. Bredwell, Prashant Srivastava, et R. Mark, dont le titre est « Reactor Design Issues for Synthesis-Gas Fermentations », publié dans la revue Biotechnology process en 1999, volume 15, pages 834-844, reconnait l'intérêt de la biométhanation du syngaz mais souligne la difficulté et l'importance de parvenir à des transferts de masses gaz-liquide suffisants pour accomplir une réaction satisfaisante de méthanation par voie biologique.

Enfin, la biométhanation du syngaz peut aussi être une réaction incomplète lorsque la dilution des gaz dans le digestat est satisfaisante mais que la flore bactérienne disponible dans l'unité de biométhanation est insuffisante en quantité ou en qualité pour traiter les quantités d'hydrogène, de monoxyde de carbone et de dioxyde de carbone effectivement dissoutes dans le milieu liquide, et que ces bactéries sont de ce fait dans l'incapacité de transformer la totalité du syngaz disponible.

Il peut résulter de ces cas de figure qu'une purification suffisante du biogaz produit soit techniquement irréalisable, la présence de monoxyde de carbone et/ou d'hydrogène résiduels rendant impossible sa transformation en un biométhane assez pur pour une injection dans un réseau de gaz naturel ou pour un usage dans un véhicule.

Certaines publications suggèrent de réaliser la transformation biologique du gaz de pyrogazéification en biogaz à partir d'une unité de méthanisation, alimentée en biomasse, dont les digestats, produits par l'unité de méthanisation, seraient séchés et envoyés dans une unité de pyrogazéification afin d'y être gazéifiés. Le gaz de pyrogazéification ainsi produit serait donc envoyé dans l'unité de méthanisation pour être transformé en biogaz. Ainsi, dans cette solution, l'unité de méthanisation de biomasse jouerait également le rôle d'unité de transformation biologique du gaz de pyrogazéification, dans laquelle celui-ci serait transformé en biogaz. En d'autres termes, cette unité serait le lieu à la fois d'une réaction de méthanisation de la biomasse et d'une réaction de transformation biologique du gaz de pyrogazéification en biogaz.

Un tel procédé réalisant à la fois la méthanisation de biomasses ordinaires et la transformation biologique du gaz de pyrogazéification brut, c'est à dire sans traitement préalable, en biogaz semble difficile à mettre en œuvre dans un usage de dimension industrielle. En effet, les matières non biodégradables, solides ou liquides, qui seraient entraînées dans le gaz de pyrogazéification, se retrouveraient dans l'unité de méthanisation, puis seraient entraînées dans les digestats produits par l'unité de méthanisation, lesquels seraient transférés dans l'unité de pyrogazéification, où ces matières non biodégradables présentes dans le gaz de pyrogazéification seraient de nouveau entraînés dans le gaz de pyrogazéification et transférées dans l'unité de méthanisation.

Ces matières non biodégradables seraient donc prisonnières d'une boucle de recirculation fermée, conduisant à une concentration croissante de celles-ci, notamment dans l'unité de méthanisation, aboutissant inévitablement à des concentrations inhibitrices pour la flore bactérienne, et finalement à une toxicité létale pour les bactéries.

En outre, dans cette configuration, l'augmentation de la concentration des matières éventuellement non biodégradables et indésirables dans l'unité de méthanisation, provenant du gaz de pyrogazéification, pourrait aussi rendre impropre à un usage agricole le digestat produit par l'unité de méthanisation. En effet, les autorisations d'épandage agricole dépendent de la concentration des matières indésirables dans les produits à épandre.

Pour résoudre cette difficulté, certaines publications suggèrent de soumettre le gaz de pyrogazéification à un prétraitement, préalablement à son traitement dans l'unité de méthanisation. Selon cette proposition, le gaz de pyrogazéification serait préalablement soumis à une opération de refroidissement, avec condensation et séparation des huiles condensées. Ces huiles, non gazéfiées, c'est-à-dire qui n'ont pas été transformées en syngaz dans l'unité de pyrogazéification, c'est-à-dire qui n'ont pas été transformées en un mélange de gaz incondensables, seraient alors retournées dans l'unité de pyrogazéification afin d'être transformées en syngaz. Finalement, selon cette proposition, l'unité méthanisation aurait pour fonction de réaliser à la fois la méthanisation de biomasses et la biométhanation d'un syngaz épuré des sous produits du gaz de pyrogazéification.

Le procédé ainsi proposé, semble lui aussi difficile à généraliser dans un usage de dimension industrielle. En effet, l'hypothèse qui préside à cette proposition est que les huiles contenues dans le gaz de pyrogazéification qui n'on pas été gazéifiées dans l'unité de pyrogazéification seront finalement gazéifiées si elles sont recirculées dans l'unité de pyrogazéification. Cette hypothèse présuppose que l'unité de pyrogazéification opère à une température suffisamment élevée pour que toutes les huiles présentes dans le gaz de pyrogazéification soient finalement susceptibles d'être gazéifiées après un certain nombre de passages dans l'unité de pyrogazéification.

Dans la réalité industrielle, la probabilité est élevée que la température à l'œuvre dans l'unité de pyrogazéification soit insuffisante pour gazéifier toutes les huiles qui n'ont pas été gazéifiées après un premier passage dans l'unité de pyrogazéification. Dans ces conditions, les huiles impropres à être gazéifiées même après plusieurs passages dans l'unité de pyrogazéification, c'est-à-dire les huiles impropres à être transformées en un mélange de gaz incondensables à la température de fonctionnement de l'unité de pyrogazéification, seraient prisonnières d'une boucle de recirculation fermée, conduisant à une accumulation croissante de celles-ci, notamment dans le gaz de pyrogazéification et dans l'unité de pyrogazéification, aboutissant inévitablement à des concentrations d'huiles telles que le procédé s'encrasserait inévitablement et deviendrait inopérant.

De plus, les bactéries anaérobies ont généralement besoin de s'acclimater aux produits qu'elles doivent digérer et de se spécialiser pour réaliser convenablement cette digestion. En effet, certains produits à digérer peuvent être inhibiteurs ou toxiques à certaines concentrations pour certaines familles de bactéries, tandis qu'ils peuvent au contraire favoriser l'activité d'autres familles de bactéries. Les réactions biologiques qui interviennent dans la méthanisation de biomasses ordinaires, ou dans la transformation biologique du gaz de pyrogazéification en biogaz, ou encore dans la biométhanation du syngaz mettent en œuvre des processus biologiques différents, qui nécessitent une adaptation des bactéries, notamment par l'acclimatation et la sélection des familles de bactéries les plus adaptées pour la digestion de chaque type de substance à traiter. Or les substances à transformer dans les biomasses ordinaires, dans le gaz de pyrogazéification et dans le syngaz sont de nature très différente. Il existe une probabilité élevée qu'une opération simultanée de transformation en biogaz de biomasses ordinaires, de sous-produits du gaz de pyrogazéification et de syngaz, donc de produits de nature très différente, dans une seule unité de méthanisation se révèle déficiente, voire inefficiente, car la flore bactérienne, présente dans l'unité unique de méthanisation, risque de présenter des difficultés à s'adapter et se spécialiser convenablement pour les trois familles de produits différentes.

En résumé, l'efficacité d'une unité réalisant à la fois la méthanisation de biomasses ordinaires, la transformation biologique du gaz de pyrogazéification en biogaz et la biométhanation du syngaz risque d'être limitée.

Enfin, les unités de pyrogazéification sont souvent des technologies difficiles à optimiser et à exploiter. En effet, de nombreuses unités de pyrogazéification rencontrent des difficultés de réglage, en particulier lorsqu'elles sont alimentées par différents types de biomasse, avec des variations sensibles de nature, de qualité ou d'humidité. De telles difficultés de réglage peuvent même subsister lorsqu'un seul type de biomasse est traité dans une unité de pyrogazéification. Par exemple, même quand la biomasse traitée correspond à des plaquettes de bois, les variations dans la qualité du bois, en particulier les variations d'humidité et de granulométrie, pyrogazéification et des variations dans la qualité du gaz produit.. <A> cf. page 15A

Ainsi les différents procédés proposés ou mis en œuvre à l'heure actuelle pour transformer les gaz de pyrogazéification, en biogaz présentent un certain nombre de difficultés.

Au vu de ce qui précède, l'invention a notamment pour objet de proposer un dispositif capable de transformer efficacement par voie biologique du gaz de pyrogazéification en biogaz, en minimisant les coûts d'investissement et d'exploitation mis en œuvre pour réaliser une telle transformation, et ceci tout en assurant une bonne fiabilité du dispositif, notamment en évitant l'affaiblissement et la dégradation de la flore bactérienne et en assurant sa bonne qualité dans le temps.

La présente invention a pour objet un dispositif (1) de transformation par voie biologique de gaz de (11) en biogaz (19, 23) comprenant :
- une unité de production de digestat liquide (3) comprenant une unité de production de digestat brut (2) et un système de séparation de phase (26), ayant ensemble pour fonction la production de digestat liquide (8) contenant des bactéries anaérobies,
- une unité fonctionnelle RLMB (4) comprenant quatre fonctions :
   <A> =

< Le document WO 2015/003273 décrit un système dans lequel un digesteur anaérobie, alimenté en biomasse, est couplé à un dispositif de pyrolyse destiné à transformer le digestat solide, produit par ledit digesteur et préalablement déshydraté, en gaz de pyrogazéification à une température comprise entre 300 et 500°C. Un tel gaz est ensuite acheminé vers une unité afin d'y être refroidi via un échangeur de chaleur et traité par un condenseur de manière à récupérer le syngaz. Ce syngaz est envoyé directement dans le digesteur anaérobie afin de produire du biogaz. Ainsi le digesteur anaérobie est le lieu d'une réaction de méthanisation de la biomasse et d'une réaction de transformation biologique du gaz de pyrogazéification en biogaz.

L'article scientifique intitulé « Intégration of pyrolisis and anaerobic digestion - Use of aqueous liquor from digestable pyrolysis for biogas production" décrit un système comprenant un digesteur anaérobie, alimenté en biomasse, produisant du digestat qui est séché avant d'être introduit dans un dispositif de pyrolyse pour conduire à du biochar, des goudrons et des gaz non condensables. La phase aqueuse des produits issus de la pyrolyse est récupérée pour être acheminée dans le digesteur anaérobie afin d'améliorer la production de biogaz. Les gaz non condensables sont quant à eux refroidis et lavés.

L'article scientifique intitulé « Pyrolysis of dried biosolids for increased biogas production" décrit un dispositif dans lequel les produits solides issus d'une unité de pyrolyse, notamment des matériaux lignocellulosiques récalcitrants n'ayant pas entièrement réagis au cours d'une digestion anaérobie, sont réacheminés vers un digesteur anaérobie afin de produire du biogaz additionnel. Ce procédé a pour but de réduire les résidus solides récalcitrants, issus de la digestion anaérobie, pour les traiter dans une unité de pyrolyse afin d'être réintroduits dans le digesteur

L'article scientifique intitulé « New opportunities for agricultural digestate valorization : current situation and perspectives » porte sur un système capable de valoriser du digestat solide produit par un dispositif anaérobie, notamment par le biais d'un procédé de pyrolyse. Le document US 2015/0118723 décrit un dispositif visant l'obtention de produits écologiques tels que du biogaz, du digestat liquide et du digestat solide. Ce dispositif comprend une unité de méthanisation capable de produire du biogaz, du digestat liquide et du digestat solide et une unité de pyrolyse, alimentée partiellement en digestat solide, capable de produire des produits huileux, du biochar et du syngaz.

Le document US 2012/0073199 décrit un procédé pour améliorer le traitement de matériaux contenant de la lignocellulose par biotraitement.>
(i) une fonction de refroidissement R du gaz de pyrogazéification (11),
(ii) une fonction de lavage L du gaz de pyrogazéification (11),
(iii) une fonction de méthanisation M d'au moins une partie des sous-produits du gaz de pyrogazéification SPGP (16b), extraits du gaz de pyrogazéification (11) par les fonctions de refroidissement R et de lavage L, afin de former du biogaz (19),
(iv) une fonction de biométhanation B du syngaz (16), extrait du gaz de pyrogazéification (11) et séparé des sous-produits du gaz de pyrogazéification SPGP (16b) par les fonctions de refroidissement R et de lavage L, afin de former du biogaz (23), l'unité fonctionnelle RLMB(4) alimentée au moins par le gaz de pyrogazéification (11), destiné à être transformé en biogaz, et par du digestat provenant, pour tout ou partie, de l'unité de production de digestat liquide (3), l'unité fonctionnelle RLMB (4) comprenant une ou plusieurs sous-unités distinctes susceptibles de réaliser chacune tout ou partie d'une ou plusieurs fonctions de refroidissement, de lavage, de méthanisation et/ou de biométhanation.

Ainsi l'unité fonctionnelle est alimentée par deux flux de matières distinctes, à savoir le gaz de pyrogazéification et du digestat provenant, pour tout ou partie, de l'unité de production de digestat liquide.

De préférence, le digestat alimentant l'unité fonctionnelle est du digestat liquide constitué, en totalité ou partie, par le digestat liquide produit par l'unité de production de digestat liquide.

Plus préférentiellement, le digestat qui alimente l'unité fonctionnelle est constitué en totalité par le digestat liquide produit par l'unité de production de digestat liquide.

De manière avantageuse, la fonction de méthanisation est alimentée au moins par du digestat provenant, pour tout ou partie, de l'unité de production de digestat liquide.

De manière plus avantageuse, la fonction de méthanisation est alimentée au moins par du digestat, lequel digestat provient en totalité de l'unité de production de digestat liquide.

De manière avantageuse, la fonction de biométhanation est alimentée au moins par du digestat provenant, pour tout ou partie, de l'unité de production de digestat liquide.

De manière plus avantageuse, la fonction de biométhanation est alimentée au moins par du digestat, lequel digestat provient en totalité de l'unité de production de digestat liquide.

L'unité de production de digestat liquide comprend une unité de production de digestat brut correspondant de préférence à une unité de méthanisation de biomasses, composée d'un ou plusieurs digesteurs anaérobies, selon un procédé en voie liquide.

L'unité de production de digestat liquide inclut également un système de séparation de phase du digestat brut produit par l'unité de production de digestat brut.

De préférence, l'unité de production de digestat liquide est une unité de méthanisation en voie liquide, c'est à dire une unité dans laquelle les biomasses à traiter, de préférence humides, sont introduites dans un ou plusieurs digesteurs anaérobies, de telle sorte que le digestat brut résultant de la digestion de ces biomasses et contenu dans le ou les digesteurs anaérobies soit aisément pompable et agitable, c'est-à-dire que ce digestat brut ait un taux de matières sèches généralement compris entre 4 et 12% en poids.

En d'autres termes, le digestat brut produit par l'unité de production de digestat brut présente de préférence un taux de matières sèches généralement compris entre 4 et 12% poids. Au sens de la présente invention, le taux de matières sèches en poids est appelé siccité.

L'unité de production de digestat liquide a deux fonctions principales.

La fonction première de l'unité de production de digestat liquide est de produire une grande quantité de digestat liquide susceptible d'alimenter l'unité fonctionnelle RLBM.

Plus précisément, la fonction première de l'unité de production de digestat liquide est d'alimenter l'unité fonctionnelle RLBM en digestat liquide en quantité suffisante pour que la concentration de chacune des substances qui composent le gaz de pyrogazéification, dans les liquides présents dans l'unité fonctionnelle, soit suffisamment faible pour permettre la bonne réalisation de chacune des fonctions de l'unité fonctionnelle RLBM:

Plus spécifiquement, il convient tout particulièrement de diluer la concentration de chacune des substances, composant le gaz de pyrogazéification, dans le digestat contenu dans la fonction de méthanisation de l'unité fonctionnelle, de façon suffisamment importante pour que chacune de ces substances se trouve dans la solution en une concentration assez faible pour rendre ladite substance non toxique pour les bactéries présentes dans ledit digestat, et non inhibitrice de l'activité de ces bactéries.

Notamment, la quantité de digestat liquide transférée depuis l'unité de production de digestat liquide vers la fonction de méthanisation est de préférence déterminée de telle façon que la substance potentiellement la plus toxique soit diluée de façon suffisante pour que cette substance soit rendue non toxique pour la flore bactérienne présente dans la fonction de méthanisation.

En effet, la plupart des substances présentes dans le gaz de pyrogazéification, et en particulier dans les sous-produits du gaz de pyrogazéification, par exemple dans les bio-huiles de bois, sont toxiques au dessus de certaines concentrations pour la majorité des familles de bactéries présentes dans le digestat agissant dans la fonction de méthanisation. A l'inverse, la plupart des substances présentes dans le gaz de pyrogazéification sont biodégradables lorsqu'elles se trouvent en solution dans le digestat en dessous de certains seuils de concentration et qu'elles sont mises en présence de bactéries adaptées à la biodégradation de ces substances.

C'est pourquoi il est très important, d'une part, de diminuer dans les digestats les concentrations des substances potentiellement toxiques ou inhibitrices, et, d'autre part, de favoriser la diversité, qui détermine le potentiel de spécialisation, et l'adaptation des familles de bactéries agissant dans les digestats des fonctions de méthanisation et de biométhanation.

La deuxième fonction de l'unité de production de digestat liquide est donc de produire un digestat liquide contenant une grande quantité de bactéries, de grande qualité, et appartenant à une grande variété de familles. C'est pourquoi le digestat liquide est de préférence obtenu à partir de la fermentation anaérobie de biomasses de bonne qualité, facilement biodégradables et dépourvues de matières toxiques ou inhibitrices. De cette façon, les bactéries se développent plus abondamment et de façon plus variée. Ainsi, l'alimentation des fonctions de méthanisation et de biométhanation par un digestat liquide abondant, contenant une grande quantité de bactéries, appartenant à des familles nombreuses et variée, a pour conséquence la production, par la compétition, la sélection naturelle et le remplacement des bactéries affaiblies, de digestats contenant des bactéries robustes, résistantes, efficaces et adaptées aux substances qu'ensemble elles transforment en biogaz.

L'unité de production de digestat liquide a pour fonctions secondaires la production de biogaz ainsi que la production de digestat solide.

Ainsi, l'unité de production de digestat liquide a pour fonctions principales de produire une grande quantité de digestat liquide susceptible de contenir une grande quantité de bactéries de bonne qualité, et appartenant à une large variété de familles, afin d'alimenter l'unité fonctionnelle en un digestat liquide susceptible de favoriser son fonctionnement.

Le dispositif selon l'invention présente l'avantage de transformer par voie biologique, de manière efficace, du gaz de pyrogazéification en biogaz, et de permettre une fiabilisation d'un tel dispositif en renouvelant, en renforçant et en améliorant continuellement la flore bactérienne présente dans l'unité fonctionnelle.

Le gaz de pyrogazéification ainsi transformé en biogaz est d'un usage plus simple et sa valorisation économique plus facile que si ce gaz avait seulement été transformé en syngaz et en sous-produits de gaz de pyrogazéification.

En effet, le biogaz présente l'avantage d'être un gaz facilement convertible en énergie renouvelable et peut notamment servir plus aisément que le syngaz à la production d'électricité et/ou de chaleur ou à la production de carburant. Le biogaz peut également être injecté dans un réseau de gaz naturel après épuration, ce qui n'est pas autorisé dans le cas d'un syngaz contenant du monoxyde de carbone.

Par exemple, le biogaz peut être valorisé par combustion dans une chaudière pour produire de la chaleur ou de l'énergie électrique au moyen d'une turbine à vapeur.

Les brûleurs de biogaz, équipant les chaudières, sont des équipements très courants et éprouvés, dont la fiabilité est désormais très bonne. Les brûleurs de biogaz peuvent être mono-combustibles ou multi-combustibles, par exemple biogaz/gaz naturel ou biogaz/fuel. Les brûleurs de syngaz sont au contraire des équipements dont la fiabilité est plus incertaine, dans la mesure où la composition du syngaz évolue facilement et souvent, en fonction des variations de quantité et de qualités des biomasses traitées. De même, le syngaz peut être encrassant pour les brûleurs, en particulier si ledit syngaz n'a pas fait préalablement l'objet d'un lavage suffisamment efficace.

Le biogaz peut aussi être valorisé par combustion dans un moteur de cogénération. Ici encore, il s'agit de technologies éprouvées, et plusieurs milliers de moteurs de cogénération ont été installés et tournent aujourd'hui au biogaz de façon très satisfaisante.

De la même façon que pour les brûleurs de chaudière, les moteurs de cogénération fonctionnant au syngaz sont moins fiables à l'usage que ceux fonctionnant au biogaz. Les risques d'encrassement ou de corrosion des métaux sont en particulier beaucoup plus élevés dans les moteurs de cogénération alimentés avec du syngaz.

Enfin, le biogaz peut être injecté dans les conduites existantes de gaz naturel ou être valorisé comme carburant pour véhicules, à la différence du syngaz qui, pour des raisons techniques et réglementaires, ne peut pas être injecté dans les tuyauteries de gaz naturel, ni être utilisé comme carburant pour véhicules.

En d'autres termes, le dispositif selon l'invention permet de mieux valoriser le gaz de pyrogazéification.

Le dispositif selon l'invention permet de transformer par voie biologique du gaz de pyrogazéification en biogaz de façon plus simple, plus fiable et moins coûteuse, en investissement et en exploitation, qu'un système de méthanation par transformation catalytique. Il permet notamment de s'affranchir de l'obligation d'installer des équipements très coûteux fonctionnant à des niveaux de pression et de température élevés.

Le dispositif selon l'invention permet aussi de résoudre un certain nombre de difficultés auxquelles font face d'autres systèmes de méthanation par voie biologique.

En effet, la présence, dans le dispositif objet de l'invention, d'une unité de production de digestat liquide permet de générer une production abondante de digestat liquide de bonne qualité pour alimenter l'unité fonctionnelle. L'alimentation de l'unité fonctionnelle avec une grande quantité de digestat liquide de bonne qualité favorise, selon la configuration retenue, certaines des quatre fonctions de l'unité fonctionnelle RLMB,

En effet, plus la quantité de biomasses traitées dans l'unité de production de digestat liquide est élevée, notamment des biomasses humides, plus la production de digestats liquides est élevée, plus la quantité de digestat liquide transférée à l'unité fonctionnelle peut être élevée, plus la quantité de digestat liquide circulant à travers l'unité fonctionnelle est importante, ce qui facilite notamment la réaction de méthanisation des sous-produits du gaz de pyrogazéification à l'œuvre dans l'unité fonctionnelle.

Le fonctionnement global de l'unité fonctionnelle se retrouve ainsi d'autant plus efficace, fiable et facilité. En outre, cette amélioration du fonctionnement global de l'unité fonctionnelle est peu coûteuse car elle est obtenue par une simple augmentation de la quantité de biomasse à traiter dans l'unité de production de digestat liquide.

L'unité de production de digestat liquide, alimentée avec de la biomasse, permet aussi de produire un digestat liquide contenant une grande quantité de bactéries variées, de bonnes qualités et en bonne santé, dans le but d'alimenter, de manière régulière, séquentielle ou de préférence continue, l'unité fonctionnelle selon l'invention.

En d'autres termes, l'unité de production de digestat liquide permet de faire circuler du digestat liquide vers l'unité fonctionnelle afin que celle-ci puisse exercer, selon la configuration retenue, certaines des quatre fonctions, telles que décrites précédemment, de manière simple, efficace et fiable.

En outre, l'unité de production de digestat liquide produit un digestat en sortie du ou des digesteurs, appelé digestat brut, comprenant une fraction solide dont le taux de matières sèches, ou siccité, est généralement compris entre 4 et 12%. Ce digestat brut est séparé en deux fractions au moyen d'un système de séparation de phase. Le système de séparation de phase produit d'une part une fraction solide, dont la siccité est généralement comprise entre 20 et 30% et une fraction liquide dont la siccité est généralement comprise entre 2 et 10%.

Le système de séparation de phase permet de séparer la fraction solide et la fraction liquide au sein du digestat brut afin de produire du digestat liquide, lequel est plus adapté que le digestat brut ou le digestat solide à l'accomplissement des fonctions de l'unité fonctionnelle. En effet, le digestat liquide possède une concentration plus faible en matières solides, et surtout, il contient des matières solides de granulométrie plus faible, ce qui le rend plus liquide et moins pâteux, et donc plus facilement pompable et agitable, et avec une propension plus faible à obturer les tuyauteries et autres dispositifs qu'il traverse.

Le digestat liquide est donc plus facile à pomper ce qui facilite sa circulation à travers l'unité fonctionnelle, et il est plus facile à agiter, notamment dans les diverses cuves qui équipent l'unité fonctionnelle. Ainsi la plus grande fluidité du digestat liquide, par rapport au digestat brut ou au digestat solide, permet une plus grande fiabilité, ainsi qu'une réduction des coûts d'investissement et d'exploitation des équipements de pompage et d'agitation qui équipent l'unité fonctionnelle.

Il est à noter que le digestat liquide, en sortie du système de séparation de phase, contient une concentration en bactéries comparable à celle du digestat brut avant séparation de phase. En effet, les matières solides présentes dans le digestat brut sont constituées d'une plus grande proportion de biomasse non dégradée et de matière inerte que les matières solides présentes dans le digestat liquide.

De préférence, le système de séparation de phase est une presse à vis. De manière alternative, le système de séparation de phase peut être mise en œuvre par tout autre technologie existante, par exemple une centrifugeuse, un filtre presse ou encore un filtre à bande.

L'alimentation en quantité importante de digestat liquide dans l'unité fonctionnelle a pour effet de diluer de préférence l'ensemble des liquides contenus dans l'unité fonctionnelle, ce qui permet notamment de diminuer les concentrations, dans le digestat présent dans la fonction de méthanisation, des substances potentiellement agressives, toxiques ou inhibitrices, vis-à-vis de la flore bactérienne, qui proviennent des sous-produits du gaz de pyrogazéification. Une telle diminution des concentrations des diverses substances agressives, toxiques ou inhibitrices, vis-à-vis de la flore bactérienne, est susceptible de favoriser l'activité, l'adaptation et donc le rendement des bactéries à l'œuvre dans la fonction de méthanisation de l'unité fonctionnelle.

La flore bactérienne, contenue dans le digestat liquide produit par l'unité de production de digestat liquide, se mélange facilement avec la flore bactérienne déjà à l'œuvre dans les fonctions de méthanisation et de biométhanation de l'unité fonctionnelle, ce qui a pour avantage de favoriser, dans ces deux fonctions, le renouvellement et la qualité des flores bactériennes utiles à la transformation du gaz de pyrogazéification en biogaz. En effet, l'alimentation en digestat liquide permet d'entretenir, voire d'augmenter la quantité de bactéries, ainsi que la diversité des familles de bactéries présentes dans les fonctions de méthanisation et de biométhanation de l'unité fonctionnelle et de remplacer les bactéries, éventuellement affaiblies ou mortes au cours de la transformation du gaz de pyrogazéification en biogaz. En effet, les sous-produits du gaz de pyrogazéification peuvent contenir des substances agressives pour les flores bactériennes. De même, le syngaz peut se révéler, à certaines doses, agressif pour certaines populations de bactérie.

Par ailleurs, le fait de séparer les trois réactions biologiques à l'œuvre dans le dispositif objet de l'invention, à savoir la réaction de méthanisation de biomasses ordinaires dans l'unité de production de digestat liquide, la réaction de méthanisation des sous-produits du gaz de pyrogazéification, et la réaction de biométhanation du syngaz conduit à une meilleure spécialisation des bactéries. En effet, les familles de bactéries à l'œuvre dans la fonction de méthanisation de l'unité fonctionnelle pourront acquérir des meilleures résistances aux substances agressives présentes dans les sous-produits du gaz de pyrogazéification, et une meilleure capacité à transformer ces substances en biogaz, les familles de bactéries à l'œuvre dans la fonction de biométhanation de l'unité fonctionnelle pourront acquérir des meilleures résistances aux fortes concentrations de syngaz, et une meilleure capacité à transformer le syngaz en biogaz, tandis que les bactéries, présentes dans l'unité de production de digestat liquide auront été renforcées par l'habitude de dégrader des biomasses simples, facilement biodégradables et exemptes de substances potentiellement toxiques, ce qui les rendra plus nombreuses, plus variées, plus robustes, plus résistantes, et donc, par sélection naturelle, mieux à même de renforcer ainsi que de rendre plus adaptées et plus efficaces les populations présentes dans l'unité fonctionnelle.

L'alimentation régulière, continue ou séquentielle, des fonctions de méthanisation et de biométhanation de l'unité fonctionnelle par du digestat liquide contenant des bactéries en provenance de l'unité de production de digestat liquide n'a pas pour effet de ralentir la spécialisation des bactéries à l'œuvre dans les fonctions de méthanisation et de biométhanation dans la mesure où les bactéries les mieux adaptées présentes dans ces deux fonctions deviendront plus nombreuses et l'emporteront sur celles des bactéries, en provenance de l'unité de production de digestat liquide, qui sont le moins adaptées à l'agressivité des matières présentes dans le gaz de pyro gazéification.

L'alimentation régulière, séquentielle ou de préférence continue, en digestat liquide de l'unité fonctionnelle RLMB, par l'unité de production de digestat liquide, est susceptible de favoriser l'accomplissement des fonctions de refroidissement et de lavage, et favorise surtout les fonctions de méthanisation des sous-produits du gaz de pyrogazéification et de biométhanation du syngaz de l'unité fonctionnelle RLBM.

Comme indiqué précédemment, l'unité fonctionnelle RLBM comprend quatre fonctions: comprenant quatre fonctions : une fonction de refroidissement R; une fonction de lavage L, une fonction de méthanisation M et une fonction de biométhanation B.

Tout d'abord, la fonction de refroidissement R de l'unité fonctionnelle permet de refroidir le gaz de pyrogazéification qui présente généralement, en sortie de l'unité de pyrogazéification, une température élevée, proche de celle mise en jeu lors des réactions de pyrogazéification, pouvant aller de 300 à 1200°C. La fonction de refroidissement facilite le traitement ultérieur du gaz de pyro gazéification.

La fonction de refroidissement peut être réalisée par tout dispositif de refroidissement existant, par exemple par un échangeur de chaleur.

De préférence, la fonction de refroidissement est réalisée par injection du gaz de pyrogazéification dans un liquide froid ou refroidi, dit liquide de refroidissement et/ou par aspersion du gaz de pyrogazéification par un tel liquide de refroidissement. De manière avantageuse, le liquide de refroidissement est constitué, pout tout ou partie, par du digestat liquide alimenté par l'unité de production de digestat liquide.

L'alimentation en digestat liquide est particulièrement avantageuse car ce dernier se trouve en abondance dans le dispositif selon l'invention. De ce fait, une augmentation de la quantité utilisée de liquide de refroidissement peut être réalisée pour un coût additionnel très limité en favorisant la production de digestat liquide dans l'unité de production de digestat liquide, notamment par l'augmentation de la quantité de biomasse humide traitée dans l'unité de production de digestat liquide.

En d'autres termes, l'unité de production de digestat liquide alimente avantageusement en digestat liquide la fonction de refroidissement de l'unité fonctionnelle.

La fonction de refroidissement est exercée par une sous-unité de refroidissement. Ainsi l'unité de production de digestat liquide alimente avantageusement en digestat liquide une sous-unité visant à refroidir le gaz de pyrogazéification dans l'unité fonctionnelle.

Par ailleurs, la fonction de refroidissement de l'unité fonctionnelle a également pour avantage d'entraîner la condensation partielle des goudrons, des huiles et des autres gaz condensables, qui se trouvent dans le gaz de pyrogazéification. Les sous-produits, devenus liquides, peuvent aussi capter, au cours de leur condensation, une partie des sous-produits solides du gaz de pyrogazéification, à savoir les particules fines solides dont des cendres, des particules de charbon et des particules minérales.

Par conséquent, au moins une partie des sous-produits issus du gaz de pyrogazéification est retenue et mélangée, voire diluée, dans le liquide de refroidissement, ce qui a pour effet de diminuer sensiblement le caractère encrassant et corrosif du gaz de pyro gazéification.

En outre, l'alimentation de la fonction de refroidissement par une grande quantité de digestat liquide favorise la baisse des concentrations dans le liquide de refroidissement des substances encrassantes, tels que les goudrons, issues des sous-produits des gaz de pyrogazéification, ce qui a pour effet de fiabiliser et faciliter l'accomplissement de la fonction de refroidissement.

Ainsi la fonction de refroidissement de l'unité fonctionnelle a également pour avantage de laver au moins partiellement le gaz de pyrogazéification en séparant une partie des sous-produits qui composent ledit gaz.

Ensuite, la fonction de lavage L de l'unité fonctionnelle RLBM permet de laver le gaz de pyrogazéification, en séparant l'ensemble des sous-produits qui n'ont pas été séparés par la fonction de refroidissement. La fonction de lavage permet ainsi d'obtenir un syngaz assez pur, composé essentiellement d'un mélange de gaz incondensables, dont la fraction en sous-produits indésirables a été minimisée.

La fonction de lavage permet donc de laver, de manière autonome ou en complément de la fonction de refroidissement, le gaz de pyrogazéification, afin d'obtenir un syngaz ayant un caractère encrassant et corrosif significativement plus faible que celui du gaz de pyrogazéification recueilli en sortie du procédé de pyrogazéification.

En d'autres termes, la fonction de lavage permet de rendre moins encrassant et moins corrosif le gaz de pyrogazéification.

En particulier, la fonction de lavage permet de séparer les sous-produits liquides du gaz de pyrogazéification et les particules fines solides. Cette fonction de lavage permet d'améliorer la pureté du syngaz, composé essentiellement de gaz incondensables, principalement du monoxyde de carbone, de l'hydrogène, du méthane et du dioxyde de carbone.

La fonction de lavage peut être réalisée par tout dispositif de lavage existant, éventuellement mécanique, thermique et/ou chimique (filtres à manche, filtres à sable, filtres céramique, cyclones, lavage à la chaux, lavage à l'huile, lavage électrostatique, traitements thermiques, etc.).

De préférence, la fonction de lavage est réalisée par injection du gaz de pyrogazéification dans un liquide froid ou refroidi, dit liquide de lavage, et/ou par aspersion du gaz de pyrogazéification par un tel liquide de lavage.

De préférence, le lavage du gaz de pyrogazéification par injection dans le liquide de lavage est avantageusement complété par une aspersion intense du gaz de pyrogazéification avec le liquide de lavage, réalisé par une douche, conformément à de nombreux procédés techniques, classiques et éprouvés, à l'œuvre dans les tours de lavage de gaz. Le liquide de lavage a préférentiellement une température comprise entre 35 et 50°C, température qui favorisera notamment la mise en œuvre de la fonction ultérieure de méthanisation des sous-produits du gaz de pyrogazéification.

Le recours à une fonction de lavage, mise en œuvre par injection du gaz dans un liquide de lavage, et/ou par une aspersion du gaz de pyrogazéification par le liquide de lavage, présente l'avantage d'être une solution simple, efficace et moins coûteuse, en termes d'investissement et d'exploitation, que les systèmes de lavage réalisés par des procédés mécaniques, thermiques ou chimiques, en particulier des technologies en voie sèche comme, par exemple, les filtres à manche, les filtres à sable, les filtres céramique, les cyclones, les dispositifs de lavage à la chaux ou les dispositifs de lavage électrostatiques, lesquels ont tendance à s'encrasser lors de leur fonctionnement et nécessitent généralement beaucoup de temps de surveillance et de maintenance.

Par ailleurs, le mélange des sous-produits du gaz de pyrogazéification avec le liquide de lavage conduit à la formation d'un déchet liquide pouvant généralement être valorisé énergétiquement par la fonction de méthanisation de l'unité fonctionnelle.

Le liquide de lavage est constitué pour tout ou partie par du digestat liquide produit par l'unité de production de digestat liquide.

Comme indiqué ci-avant, l'alimentation par du digestat liquide de la fonction de lavage est particulièrement avantageuse car le digestat liquide se trouve en abondance dans le dispositif selon l'invention. De ce fait, une augmentation de la quantité du liquide de lavage peut être réalisée pour un coût additionnel très limité en favorisant simplement la production de digestat liquide dans l'unité de production de digestat liquide, notamment par l'augmentation de la quantité de biomasse humide traitée dans l'unité de production de digestat liquide.

En outre, l'alimentation de la fonction de lavage par une grande quantité de digestat liquide favorise la baisse des concentrations dans le liquide de lavage des substances encrassantes, tels que les goudrons, issues des sous-produits des gaz de pyrogazéification, ce qui a pour effet de fiabiliser et faciliter l'accomplissement de la fonction de lavage.

En d'autres termes, l'unité de production de digestat liquide alimente avantageusement en digestat liquide la fonction de lavage de l'unité fonctionnelle RLBM.

La fonction de lavage est exercée par une sous-unité de lavage. Ainsi l'unité de production de digestat liquide alimente de manière avantageuse en digestat liquide une sous-unité visant à laver le gaz de pyrogazéification dans l'unité fonctionnelle RLBM.

Par ailleurs, la fonction de méthanisation permet de réaliser une réaction de méthanisation, par voie biologique, dans des conditions anaérobies, des sous-produits du gaz de pyrogazéification, lesquels ont été séparés du syngaz par les fonctions de refroidissement et de lavage de l'unité fonctionnelle, en vue d'être transformés en biogaz.

En particulier, la fonction de méthanisation M permet de valoriser les sous-produits du gaz de pyrogazéification, initialement issus du gaz de pyrogazéification, en transformant ces sous-produits en biogaz, essentiellement constitué de méthane et de monoxyde de carbone.

La fonction de méthanisation permet de valoriser le mélange des sous-produits du gaz de pyrogazéification et d'un liquide de lavage, lequel liquide est de préférence constitué pour tout ou partie du digestat liquide alimenté par l'unité de production de digestat liquide, en transformant ces sous-produits en biogaz.

La fonction de méthanisation permet en outre de transformer en biogaz les sous-produits issus du gaz de pyrogazéification après qu'ils ont été introduits dans le digestat présent dans ladite fonction de méthanisation. Ces sous-produits sont donc dégradés par l'action de bactéries contenues dans le digestat, lui-même contenu dans la fonction méthanisation.

Le digestat contenu dans la fonction méthanisation provient, pour tout ou partie, du digestat liquide produit par l'unité de production de digestat liquide.

De manière plus avantageuse, le digestat contenu dans la fonction méthanisation provient en totalité du digestat liquide produit par l'unité de production de digestat liquide.

L'alimentation en digestat liquide de la fonction de méthanisation de l'unité fonctionnelle RLBM, par l'unité de production de digestat liquide, permet notamment de diminuer la concentration de chacune des substances qui composent les sous-produits du gaz de pyrogazéification dans le digestat contenu dans la fonction de méthanisation, de façon suffisamment importante pour que chacune de ces substances se trouve dans la solution à une concentration assez faible pour rendre ladite substance non toxique pour les bactéries présentes dans le digestat, et non inhibitrice de l'activité de ces bactéries.

En outre, le maintien d'une faible concentration de chacune des substances qui composent les sous-produits du gaz de pyrogazéification, dans le digestat contenu dans la fonction de méthanisation, permet aux bactéries de pouvoir dégrader plus efficacement la majorité de ces substances, ce qui favorise la production de biogaz issu de l'unité de méthanisation.

De plus, l'alimentation en digestat liquide de la fonction de méthanisation de l'unité fonctionnelle RLBM, par l'unité de production de digestat liquide, permet de renforcer ou de remplacer les bactéries qui sont affaiblies ou qui disparaissent au cours de la réaction de méthanisation. Elle permet aussi d'augmenter la quantité et la variété des familles de bactéries à l'œuvre dans la réaction biologique de méthanisation.

Ainsi cette alimentation permet d'augmenter la quantité, la variété, et donc, par sélection naturelle, la qualité, la résistance, l'adaptation et l'efficacité des bactéries responsables de la méthanisation des sous-produits du gaz de pyrogazéification.

L'unité de production de digestat liquide alimente donc avantageusement en digestat liquide la fonction de méthanisation de l'unité fonctionnelle RLBM.

La fonction de méthanisation est exercée par une sous-unité de méthanisation.

Ainsi l'unité de production de digestat liquide alimente de manière avantageuse en digestat liquide une sous-unité visant à méthaniser les sous-produits issus du gaz de pyrogazéification dans l'unité fonctionnelle.

En particulier, la sous-unité de méthanisation est constituée d'un ou plusieurs digesteurs anaérobies.

Ainsi selon un mode de réalisation, la fonction de méthanisation est une sous-unité de méthanisation, constituée d'un ou plusieurs digesteurs anaérobies, avantageusement alimentée par du digestat liquide produit par l'unité de digestat liquide.

Enfin, la fonction de biométhanation permet de réaliser une réaction de méthanation B par voie biologique, ou biométhanation, dans des conditions anaérobies, du syngaz, lequel a été séparé des sous-produits du gaz de pyrogazéification par les fonctions de refroidissement et de lavage, en vue de transformer ledit syngaz en biogaz, essentiellement constitué de méthane et de monoxyde de carbone.

En particulier, la fonction de biométhanation réalise la transformation du syngaz en biogaz en deux étapes : une première étape de dissolution du syngaz dans du digestat afin de mettre en contact intime le syngaz et les bactéries présentes dans ce digestat, et une deuxième étape de transformation effective du syngaz en biogaz, par l'action des bactéries présentes dans le digestat.

De manière avantageuse, le digestat contenu dans la fonction biométhanation provient, pour tout ou partie, du transfert du digestat liquide produit par l'unité de production de digestat liquide.

De manière plus avantageuse, le digestat contenu dans la fonction biométhanation provient en totalité du transfert du digestat liquide produit par l'unité de production de digestat liquide.

L'alimentation en digestat liquide de la fonction de biométhanation de l'unité fonctionnelle RLMB par l'unité de production de digestat liquide permet de renforcer ou de remplacer les bactéries qui sont affaiblies ou qui disparaissent au cours de la réaction de biométhanation. Elle permet aussi d'augmenter la quantité et la variété des familles de bactéries à l'œuvre dans la réaction biologique de biométhanation, et donc, par sélection naturelle, d'augmenter leur robustesse, leur résistance, leur efficacité et leur adaptation aux gaz incondensables qu'ensemble elles transforment en biogaz.

Ainsi cette alimentation permet d'augmenter la quantité, la variété, et donc, par sélection naturelle, la qualité, la résistance, l'adaptation et l'efficacité des bactéries responsables de la biométhanation du syngaz.

La fonction de biométhanation est exercée par une sous-unité de biométhanation, composée d'un ou plusieurs digesteurs anaérobie.

Ainsi l'unité de production de digestat liquide alimente de manière avantageuse en digestat liquide une sous-unité visant à réaliser la biométhanation du syngaz afin d'obtenir du biogaz.

En particulier, la sous-unité de biométhanation est constituée d'un ou plusieurs digesteurs anaérobies.

Ainsi selon un mode de réalisation, la fonction de biométhanation est une sous-unité de biométhanation, constituée d'un ou plusieurs digesteurs anaérobies, alimentée par du digestat liquide produit par l'unité de digestat liquide.

Il en résulte que le dispositif selon l'invention permet de traiter le gaz de pyrogazéification au moyen des quatre fonctions de l'unité fonctionnelle dans le but de le transformer en biogaz de manière efficace, fiable et durable.

En particulier, au sein du dispositif selon l'invention, l'unité de production de digestat liquide alimente avantageusement l'unité fonctionnelle en quantité abondante de digestat liquide de grande qualité.

En particulier, l'unité de production de digestat liquide produit une grande quantité de digestat liquide, ce qui permet à la fonction de refroidissement de disposer éventuellement d'une grande quantité de liquide de refroidissement, et à la fonction de lavage de disposer éventuellement d'une grande quantité de liquide de lavage. Une telle abondance de liquides de refroidissement et de lavage est susceptible de diluer les concentrations des matières encrassantes dans les liquides de refroidissement et de lavage, ce qui fiabilise et facilite l'accomplissement des fonctions de refroidissement et de lavage.

La production d'une grande quantité de digestat liquide par l'unité de production de digestat liquide permet aussi à la fonction de méthanisation de disposer éventuellement d'une grande quantité de digestat liquide.

Une telle abondance de digestat liquide favorise la dilution des matières potentiellement toxiques dans le digestat agissant dans la fonction de méthanisation, ce qui est susceptible de favoriser la fonction de méthanisation.

En outre, l'unité de production de digestat liquide produit une grande quantité de digestat liquide, lequel digestat liquide contient une grande quantité de bactéries robustes, variées et de bonne qualité, dans le but d'alimenter les fonctions de méthanisation et de biométhanation, ce qui permet de renouveler et renforcer efficacement la flore bactérienne présente dans ces deux fonctions, ce qui a pour effet d'augmenter la quantité, la variété, et donc la qualité, la résistance, l'adaptation et l'efficacité des bactéries responsables des fonction de méthanisation et de biométhanation.

Ainsi, l'alimentation de l'unité fonctionnelle RLBM en digestat liquide en provenance de l'unité de production de digestat liquide permet de rendre plus fiables et plus faciles à mettre en œuvre au moins les deux fonctions de méthanisation et de biométhanation de l'unité fonctionnelle, et permet, de préférence, de rendre plus fiables, plus efficaces et plus faciles à mettre en œuvre les quatre fonctions à l'œuvre dans l'unité fonctionnelle RLBM.

L'unité fonctionnelle RLBM comprend une ou plusieurs sous-unités distinctes susceptibles de réaliser chacune tout ou partie d'une ou plusieurs des fonctions de refroidissement, de lavage, de méthanisation et/ou de biométhanation.

Ainsi l'unité fonctionnelle RLBM peut être constituée de quatre, ou de trois, d'une seule ou de deux sous-unités distinctes susceptibles de réaliser ensemble les quatre fonctions de refroidissement, de lavage, de méthanisation et de biométhanation.

Dans les divers modes de réalisation selon l'invention, la ou les sous-unités qui réalisent, au sein de l'unité fonctionnelle RLBM, les fonctions de refroidissement et/ou de lavage sont alimentées, de préférence, par du digestat liquide produit par l'unité de production de digestat liquide.

Dans les divers modes de réalisation selon l'invention, la ou les sous-unités qui réalisent, au sein de l'unité fonctionnelle RLBM, les fonctions de méthanisation et/ou de biométhanation sont alimentées par du digestat liquide produit par l'unité de production de digestat liquide.

Selon un mode de réalisation préféré, les fonctions de refroidissement et de lavage sont réalisées par la même sous-unité.

Dans ce cas, les deux fonctions de refroidissement et de lavage sont réalisées de préférence par injection du gaz de pyrogazéification dans un liquide froid ou refroidi, dit liquide de refroidissement et de lavage, et/ou par aspersion du gaz de pyrogazéification par un tel liquide de refroidissement et de lavage, constitué de préférence, pour tout ou partie, par du digestat liquide produit par l'unité de production de digestat liquide.

Dans un tel mode de réalisation, les deux fonctions de refroidissement et de lavage sont réalisées simultanément.

En d'autres termes, dans un mode de réalisation préféré, l'unité fonctionnelle comprend une sous-unité susceptible de réaliser à la fois la fonction de refroidissement et la fonction de lavage du gaz de pyro gazéification.

Dans un mode de réalisation plus préféré, l'unité fonctionnelle comprend une sous-unité susceptible de réaliser à la fois la fonction de refroidissement et la fonction de lavage, une sous-unité capable de réaliser la fonction de méthanisation et une sous-unité susceptible de réaliser la fonction de biométhanation.

Ainsi, conformément à ce mode de réalisation préféré, l'unité de production de digestat liquide produit du digestat liquide afin d'alimenter une sous-unité de refroidissement et de lavage, une sous-unité méthanisation et une sous-unité de biométhanation.

Selon un autre mode de réalisation, l'unité fonctionnelle comprend au moins quatre sous-unités distinctes susceptibles de réaliser chacune principalement la fonction de refroidissement, de lavage, de méthanisation et de méthanation biologique définies ci-avant.

Ainsi chaque sous-unité de l'unité fonctionnelle réalise en priorité une seule fonction.

En d'autres termes, l'unité fonctionnelle comprend une sous-unité de refroidissement, une sous-unité de lavage, une sous-unité de méthanisation et une sous-unité de biométhanation.

Ainsi, conformément à ce mode de réalisation, l'unité de production de digestat liquide produit du digestat liquide afin d'alimenter au moins une sous-unité de méthanisation et une sous-unité de biométhanation.

Selon ce mode de réalisation, la sous-unité de refroidissement est, de préférence, alimentée par du digestat liquide en provenance de l'unité de production de digestat liquide, de même que la sous-unité de lavage est, elle aussi, de préférence, alimentée par du digestat liquide en provenance de l'unité de production de digestat liquide.

Selon un autre mode de réalisation, les fonctions de lavage et de méthanisation sont réalisées par la même sous-unité.

Dans ce cas, le gaz de pyrogazéification, préalablement refroidi dans une sous-unité de refroidissement, et dont les matières condensées ont été partiellement séparées ou non, est injecté dans une sous-unité apte à réaliser à la fois les deux fonctions de lavage et de méthanisation. Plus précisément, le gaz de pyrogazéification, préalablement refroidi dans une unité de refroidissement, et dont les matières condensées ont été partiellement séparées ou non, est injecté dans du digestat, lequel digestat provient, pour tout ou partie, du transfert du digestat liquide produit par l'unité de production de digestat liquide.

En d'autres termes, dans ce mode de réalisation, l'unité fonctionnelle comprend une sous-unité susceptible de réaliser la fonction de refroidissement, une sous-unité susceptible de réaliser à la fois la fonction de lavage et la fonction de méthanisation et une sous-unité susceptible de réaliser la fonction de biométhanation.

Ainsi, conformément à ce mode de réalisation, l'unité de production de digestat liquide produit du digestat liquide afin d'alimenter au moins une sous-unité de lavage et de méthanisation, et une sous-unité de biométhanation.

Selon ce mode de réalisation, la sous-unité de refroidissement est, elle aussi, de préférence, alimentée par du digestat liquide en provenance de l'unité de production de digestat liquide.

Il est à noter que dans ce mode de réalisation la fonction de méthanisation et la fonction de biométhanation s'exercent non plus en parallèle mais en série, c'est-à-dire l'une à la suite de l'autre, dans la mesure où la sous-unité de lavage et de méthanisation produit un mélange de syngaz, issu de la fonction de lavage, et de biogaz, issu de la fonction de méthanisation. Dans ce mode de réalisation, le gaz qui est injecté dans la sous-unité de biométhanation pour être transformé en biogaz est donc un mélange de syngaz et de biogaz, lequel mélange provient de la sous-unité de lavage et de méthanisation.

Selon un mode de réalisation les fonctions de refroidissement, de lavage et de méthanisation sont réalisées par la même sous-unité.

En d'autres termes, dans ce mode de réalisation, l'unité fonctionnelle RLBM comprend une sous-unité susceptible de réaliser à la fois la fonction de refroidissement, la fonction de lavage et la fonction de méthanisation et une sous-unité susceptible de réaliser la fonction de biométhanation.

Ainsi, conformément à ce mode de réalisation, l'unité de production de digestat liquide produit du digestat liquide afin d'alimenter au moins une sous-unité de refroidissement, de lavage et de méthanisation, et une sous-unité de biométhanation.

Selon un autre mode de réalisation, les fonctions de méthanisation et de biométhanation sont réalisées par la même sous-unité.

Selon un autre mode de réalisation, les fonctions de refroidissement, de lavage, de méthanisation et de biométhanation sont réalisées par la même sous-unité.

Par ailleurs, l'unité de production de digestat liquide produit, en plus du digestat liquide, un digestat solide ayant généralement une siccité pouvant être comprise entre 20 et 30%.

Le digestat solide correspond à la fraction solide du digestat brut traité par le système de séparation de phase.

Selon un mode de réalisation, le dispositif selon l'invention comprend en outre une unité de séchage de la fraction solide du digestat, produit par l'unité de production de digestat liquide, pour sécher partiellement ou complètement le digestat solide.

Selon un mode de réalisation, le dispositif selon l'invention comprend en plus de l'unité de séchage, une unité de broyage du digestat solide produit par l'unité de production de digestat liquide, pour broyer partiellement ou complètement le digestat solide. Cette unité de broyage peut se situer en amont ou en aval de l'unité de séchage du digestat solide.

Selon un mode de réalisation, le dispositif selon l'invention comprend en plus des unités de séchage et éventuellement de broyage, une unité de granulation du digestat solide produit par l'unité de production de digestat liquide, afin de granuler tout ou partie du digestat solide. Cette unité de granulation se situe en aval du l'unité de séchage et en aval de l'éventuelle unité de broyage du digestat solide.

Selon un mode de réalisation, le dispositif selon l'invention permet d'alimenter une unité de pyrogazéification par tout ou partie du digestat solide issu du dispositif, laquelle partie du digestat solide peut avoir été préalablement partiellement ou complètement séché, partiellement ou complètement broyé, et partiellement ou complètement granulé.

De préférence, l'unité de pyrogazéification qui est alimentée par le digestat solide issu du dispositif, est la même unité de pyrogazéification que celle qui produit les gaz de pyrogazéification qui sont transformés en biogaz dans le dispositif objet de l'invention.

L'unité de production de digestat liquide présente l'avantage de produire un digestat solide homogène et de qualité constante, en raison du temps de séjour long de la biomasse dans l'unité de production de digestat liquide, souvent supérieur à 30 jours. En outre, le système de séparation de phases, de part la constance de son fonctionnement, produit des digestats solides dont la siccité, c'est à dire le taux de matières sèche, est généralement constante.

L'alimentation de l'unité de pyrogazéification par du digestat solide, de qualité et de siccité constante, seul ou en mélange avec d'autres biomasses à valoriser, a donc pour effet d'augmenter la stabilité du fonctionnement de l'unité de pyrogazéification, ce qui facilite son exploitation et stabilise la production de gaz de pyrogazéification, et qui facilite en conséquence l'exploitation du dispositif objet de la présente invention.

Par ailleurs, l'unité de production de digestat liquide peut éventuellement être installée sur un site où sont déjà installés un ou plusieurs digesteurs de méthanisation de déchets d'origine agricole, ou d'origine industrielle ou encore d'origine urbaine. Dans ce cas, l'unité de production de digestat liquide objet de l'invention peut être avantageusement couplée avec le digesteur de méthanisation existant afin de favoriser les synergies entre les deux installations. Par exemple, l'unité de production de digestat liquide peut partager avec l'installation existante de méthanisation de déchets d'origine agricole, ou d'origine industrielle ou encore d'origine urbaine, les équipements d'échanges ou de transfert de chaleur, ou les équipements de stockage ou de valorisation du biogaz, ou les équipement électriques ou électroniques de conduite de l'installation, ou les équipements de préparation de la biomasse avant introduction dans les digesteurs respectifs, ou encore les équipements de préparation et de valorisation des digestats solides produits par les digesteurs respectifs (séchage, épandage agricole etc.).

En revanche, de préférence, il n'y aura pas d'échange de digestat entre les deux digesteurs. En particulier il n'y aura pas de transfert de digestat depuis le digesteur existant de déchets d'origine agricole, ou d'origine industrielle ou d'origine urbaine vers l'unité de production de digestat liquide, dans la mesure où l'unité de production de digestat liquide a pour objectif principal de produire un digestat liquide de haute qualité bactériologique et qu'un transfert de digestat depuis le digesteur agricole, ou industriel, ou urbain préexistant vers l'unité de production de digestat liquide pourrait altérer la robustesse, la résistance, la santé, la qualité et la variété des bactéries présentes l'unité de production de digestat liquide.

La présente invention a également pour objet un procédé de transformation par voie biologique du gaz de pyrogazéification (11) en biogaz (19, 23) comprenant :
- une étape d'alimentation d'une unité focntionelle RLMB (4) telle que définie précédemment, par du digestat contenant des bactéries anaérobies, lequel digestat est constitué au moins en partie par le digestat liquide (8) produit par une unité de production de digestat liquide (3) telle que définie précédemment,
- une étape de transfert du gaz de pyrogazéification vers l'unité fonctionelle RLMB (4), puis
- une étape de refroidissement du gaz de pyrogazéification (11),
- une étape de lavage du gaz de pyrogazéification (11), pour séparer, d'une part, le syngaz (16) et, d'autre part, les sous-produits du gaz de pyrogazéification SPGP (16b),
- une étape de méthanisation par voie biologique d'au moins une partie des sous-produits, extraits du gaz de pyrogazéification (11) lors des étapes refroidissement et de lavage, pour produire au moins du biogaz (23),
- une étape de méthanation par voie biologique, ou biométhanation, du syngaz (16), extrait du gaz de pyrogazéification (11) lors des étapes de refroidissement et de lavage, pour produire au moins du biogaz (23).

Le procédé selon l'invention permet de transformer de manière simple, efficace et fiable du gaz de pyrogazéification en biogaz par voie biologique, notamment en diminuant les concentrations des substances potentiellement toxiques pour la flore bactérienne dans l'étape de méthanisation, et en évitant l'affaiblissement et la dégradation de la flore bactérienne et en assurant dans le temps sa quantité, sa variété, sa qualité, sa résistance et son efficacité dans les étapes de méthanisation et de biométhanation.

L'étape de transfert du gaz de pyrogazéification vers l'unité fonctionnelle RLMB et l'étape d'alimentation en digestat liquide de l'unité fonctionnelle RLMB sont de préférence mises en œuvre en parallèle de façon régulière, continue et simultanée.

Les étapes de refroidissement, de lavage, de méthanisation et de biométhanation sont mises en œuvre après l'étape de transfert du gaz de pyrogazéification vers l'unité fonctionnelle RLBM.

Les étapes de refroidissement, de lavage, de méthanisation et de biométhanation sont réalisées au sein de l'unité fonctionnelle selon l'invention et sont mises en œuvre respectivement par les fonctions de refroidissement, de lavage, de méthanisation et de biométhanation de l'unité fonctionnelle précédemment décrite.

De préférence, l'étape de refroidissement est mise en œuvre par injection du gaz de pyrogazéification dans un liquide de refroidissement et/ou par aspersion du gaz de pyrogazéification par un liquide de refroidissement. En particulier, le liquide de refroidissement est de préférence constitué, pour tout ou partie, par le transfert du digestat liquide produit par l'unité de production de digestat liquide lors de l'étape d'alimentation.

Comme indiqué précédemment, l'étape de refroidissement permet d'abaisser la température du gaz de pyrogazéification en sortie du procédé de gazéification.

De préférence, l'étape de lavage est mise en œuvre par injection du gaz de pyrogazéification dans un liquide de lavage et/ou par aspersion du gaz de pyrogazéification par un liquide de lavage.

Préférentiellement, l'étape de lavage est mise en œuvre par injection du gaz de pyrogazéification dans un liquide de lavage et elle est complétée par l'aspersion du gaz de pyrogazéification par un liquide de lavage.

Le liquide de lavage a préférentiellement une température comprise entre 35 et 50°C, température susceptible de favoriser la mise en œuvre de l'étape ultérieure de méthanisation.

En particulier, le liquide de lavage est de préférence constitué, pour tout ou partie, par le transfert du digestat liquide produit par l'unité de production de digestat liquide lors de l'étape d'alimentation.

Comme indiqué précédemment, l'étape de lavage permet de minimiser le caractère encrassant et corrosif du gaz de pyrogazéification, en séparant, d'une part, les sous-produits du gaz de pyrogazéification, qui, de préférence, sont mis en solution dans un liquide, et d'autre part, un mélange de gaz incondensables, appelé syngaz.

Le syngaz obtenu présente un degré de pureté plus élevé que celui du gaz de pyrogazéification obtenu en sortie du ou des unités de gazéification car les sous-produits, responsables du caractère encrassant et corrosif du gaz de pyrogazéification, ont été séparées et de préférence mis en solution dans un liquide.

De préférence, l'étape de méthanisation est mise en œuvre par une sous-unité de méthanisation, constituée en particulier d'un ou plusieurs digesteurs anaérobies, qui agit notamment en transformant en biogaz les sous-produits, issus des étapes de refroidissement et de lavage, par l'action des bactéries contenues dans du digestat.

Ainsi les sous-produits, issus des étapes de refroidissement et de lavage du gaz de pyrogazéification, circulent vers une sous-unité de méthanisation de l'unité fonctionnelle RLBM.

Par ailleurs, le digestat présent dans la sous-unité de méthanisation provient, pour tout ou partie, du transfert du digestat liquide produit par l'unité de production de digestat liquide lors de l'étape d'alimentation.

De préférence, le digestat présent dans la sous-unité de méthanisation provient en totalité du transfert du digestat liquide produit par l'unité de production de digestat liquide lors de l'étape d'alimentation.

Comme indiqué précédemment, l'étape de méthanisation permet de valoriser les sous-produits issus de l'étape de lavage du gaz de pyrogazéification afin de les transformer en biogaz.

De préférence, l'étape de biométhanation du syngaz est mise en œuvre par une sous-unité de biométhanation, constituée en particulier d'un ou plusieurs digesteurs anaérobies, qui agit notamment en dissolvant le syngaz dans du digestat et en le transformant, au moyen des bactéries présentes dans ce digestat, en un biogaz. Ainsi le syngaz, obtenu après l'étape de lavage, circule vers une sous-unité de biométhanation de l'unité fonctionnelle RLBM.

En particulier, le digestat présent dans la sous-unité de biométhanation provient, pour tout ou partie, du transfert du digestat liquide produit par l'unité de production de digestat liquide lors de l'étape d'alimentation.

De préférence, le digestat présent dans la sous-unité de biométhanation provient en totalité du transfert du digestat liquide produit par l'unité de production de digestat liquide lors de l'étape d'alimentation.

Les étapes du procédé intervenant dans l'unité fonctionnelle RLMB peuvent être mises en œuvre par une ou plusieurs sous-unités distinctes de l'unité fonctionnelle

Comme décrit précédemment, les diverses étapes de refroidissement, de lavage, de méthanisation et/ou de biométhanation peuvent être mises en œuvre par quatre, ou trois, ou une seule, ou deux sous-unités distinctes de l'unité fonctionelle RLMB.

De même, chaque sous-unité peut mettre en œuvre totalement ou partiellement une, ou deux, ou trois ou quatre étapes de manière simultanée.

Ainsi, selon divers modes de réalisation, l'unité fonctionnelle RLMB peut être constituée de quatre, ou de trois, ou d'une seule ou de deux sous-unités distinctes susceptibles de réaliser ensemble les quatre fonctions de refroidissement, de lavage, de méthanisation et de biométhanation.

Selon un mode de réalisation, l'étape de refroidissement, et éventuellement l'étape de lavage, sont mises en œuvre par échange de chaleur sans injection du gaz de pyrogazéification dans un liquide, et sans aspersion de ce gaz par un liquide, par exemple au moyen d'un échangeur de chaleur. Cette étape de refroidissement du gaz de pyrogazéification s'accompagne d'une condensation d'au moins une partie des gaz condensables présents dans le gaz de pyrogazéification, lesquels gaz condensables peuvent entraîner au moins une partie des matières solides présentes dans le gaz de pyrogazéification.

Selon un mode réalisation, le procédé selon l'invention comprend une étape de séparation d'au moins une partie des sous-produits, issues de l'étape de refroidissement et éventuellement de l'étape de lavage du gaz de pyrogazéification, afin que ces sous-produits séparés, liquides et éventuellement solides, ne soient pas incluse dans l'étape de méthanisation de l'unité fonctionnelle RLBM.

Selon un mode de réalisation, le procédé comprend une étape de séparation d'une partie des sous-produits du gaz de pyrogazéification au cours de l'étape de refroidissement et/ou de lavage avant l'étape de méthanisation.

L'étape de séparation peut notamment avantageusement être mise en œuvre lorsqu'au moins une partie des sous-produits contenus dans le gaz de pyrogazéification est non biodégradable ou bien quand la dilution de ces sous-produits dans le digestat de l'unité de méthanisation est insuffisante pour évité une toxicité pour la flore bactérienne à l'œuvre dans l'étape de méthanisation. Dans cette circonstance, la partie non biodégradable ou toxique des sous-produits contenus dans le gaz de pyrogazéification doit être séparée afin que cette partie ne soit pas soumise à l'étape de méthanisation de l'unité fonctionelle RLMB.

En effet, certains sous-produits contenus dans le gaz de pyrogazéification, en particulier certains sous-produits condensables issus de la pyrogazéification de certaines matières plastiques, peuvent se révéler non biodégradables et/ou toxiques vis-à-vis de la flore bactérienne anaérobie, notamment vis-à-vis des bactéries intervenant au cours de la réaction biologique de méthanisation à l'œuvre dans l'étape de méthanisation.

De tels sous-produits peuvent donc altérer le déroulement de la réaction de méthanisation ainsi que le rendement de la production de biogaz durant l'étape de méthanisation de l'unité fonctionnelle RLBM.

Cette étape de séparation d'une partie des sous-produits du gaz de pyrogazéification au cours de l'étape de refroidissement et/ou de lavage afin que les sous-produits séparés ne soient pas transférés vers l'étape de méthanisation, sera dénommée l'étape de séparation.

Par conséquent, l'étape de séparation de tout ou partie des sous-produits du gaz de pyrogazéification est avantageusement mise en œuvre pour séparer les sous-produits toxiques présents parmi les sous-produits du gaz de pyrogazéification, issus de l'étape de refroidissement et ou de lavage, afin de favoriser la réaction de méthanisation à l'œuvre dans l'étape de méthanisation de l'unité fonctionnelle RLBM.

En outre, l'étape de séparation peut être mise en œuvre pour des raisons agronomiques.

En effet, les digestats excédentaires produits par la fonction de méthanisation ont, de préférence, comme débouché naturel l'épandage agricole.

Toutefois, afin que les digestats soient utilisables comme amendements agricoles, ces derniers ne doivent pas être contaminés ni être impropres à l'épandage sur des terres agricoles, en particulier ils ne doivent ni être contaminés par des concentrations trop élevées en produits toxiques pour les végétaux ou les animaux ou qui risqueraient de rendre toxiques, pour la consommation humaine, les végétaux produits sur ces terres ou les animaux qui auraient ingéré ces végétaux, ni être contaminants pour les terres agricoles, les eaux de ruissèlement ou les nappes phréatiques.

Par conséquent, l'étape de séparation peut se révéler nécessaire pour permettre la production de digestats moins contaminants afin qu'ils puissent être utilisés convenablement pour l'épandage agricole.

Par ailleurs, l'étape de séparation peut aussi parfois présenter l'avantage de permettre la récupération d'une partie des sous-produits, issus de l'étape de refroidissement et ou de lavage du gaz de pyrogazéification, ce qui, dans certains cas offre l'opportunité de permettre de valoriser ces sous-produits autrement, parfois de façon plus favorable que de façon énergétique par le biais de la fonction de méthanisation.

Par exemple, l'étape de séparation peut permettre une récupération avantageuse de sous-produits ayant une valeur ajoutée supérieure dans un usage industriel autre qu'énergétique, en particulier pour des utilisations dans l'industrie chimique, pharmaceutique ou cosmétique.

Selon un mode de réalisation, le procédé selon l'invention comprend une étape de dilution des matières solides et condensables, issues des étapes de refroidissement et de lavage du gaz de pyrogazéification, avant leur transfert vers la fonction de méthanisation de l'unité prodige.

L'étape de dilution peut être mise en œuvre en injectant une plus grande quantité de digestat liquide, en provenance de l'unité de production de digestat liquide, dans la sous-unité de méthanisation.

Cette étape permet de diluer les sous-produits, issus des étapes de refroidissement et de lavage du gaz de pyrogazéification, afin de réduire leur caractère potentiellement toxique pour les bactéries intervenant au cours de l'étape de méthanisation.

De manière alternative ou complémentaire, les étapes de refroidissement et de lavage peuvent être mises en œuvre en utilisant une plus grande quantité de liquide de refroidissement ou de lavage de manière à diluer davantage les sous-produits issus du gaz de pyrogazéification.

L'unité de production de digestat liquide alimente en digestat liquide au moins les fonctions de méthanisation et de biométhanation de l'unité fonctionnelle.

Selon un mode de réalisation, l'unité de production de digestat liquide alimente en digestat liquide chacune des fonctions de refroidissement, de lavage, de méthanisation et de biométhanation de l'unité fonctionnelle.

De préférence, l'unité de production de digestat liquide alimente en digestat liquide une sous-unité de refroidissement et de lavage, une sous-unité de méthanisation et une sous-unité de biométhanation.

En particulier, dans ce mode de réalisation, l'unité de production de digestat liquide alimente abondamment en digestat liquide, de manière régulière, continue ou séquentielle, les fonctions de refroidissement, de lavage, de méthanisation et de biométhanation de l'unité fonctionnelle.

L'alimentation régulière et abondante en digestat liquide des différentes fonctions de l'unité fonctionnelle est favorisée par la quantité de biomasse, notamment de biomasse très humide, traitée par l'unité de production de digestat liquide.

En effet, plus la quantité de biomasse traitée par l'unité de production de digestat liquide est élevée, plus la quantité de digestats liquides, intervenant dans les différentes étapes qui ont lieu dans l'unité fonctionelle RLBM, peut être également élevée.

Ainsi l'augmentation de la quantité de biomasse traitée par l'unité de production de digestat liquide, en particulier de la biomasse humide, conduit à une augmentation de la quantité de digestat liquide produite et peut permettre d'alimenter en plus grande quantité les différentes fonctions de l'unité fonctionnelle RLBM.

Dans un mode de réalisation, l'augmentation de l'alimentation en digestats liquide de la fonction de lavage permet notamment de diluer davantage les sous-produits issus du gaz de pyrogazéification ce qui diminue leurs concentrations ainsi que leur caractère corrosif et d'encrassement.

De même, en ce qui concerne l'étape de méthanisation, l'alimentation régulière et abondante de digestat liquide favorise le maintien de faibles concentrations en sous-produits de gaz de pyrogazéification dans les digestats qui agissent dans l'étape de méthanisation.

Une telle alimentation permet en effet de diluer les digestats qui agissent dans l'étape de méthanisation, et permet donc de diminuer la concentration des substances agressives et potentiellement toxiques vis-à-vis de la flore bactérienne intervenant dans l'étape de méthanisation. En ce qui concerne l'étape de méthanisation et l'étape de biométhanation, l'alimentation de manière régulière et abondante en digestat liquide des fonctions de méthanisation et de biométhanation permet d'augmenter la quantité, la variété, la qualité, la résistance et l'efficacité des bactéries intervenant lors des réactions de méthanisation et de biométhanation. En effet, l'alimentation régulière et abondante en digestat liquide permet d'entretenir la diversité des familles de bactéries intervenant lors des réactions de méthanisation et de biométhanation et de remplacer les bactéries, éventuellement affaiblies ou mortes, au cours de ces réactions mises en œuvre dans les étapes de méthanisation et de biométhanation.

D'autres avantages et caractéristiques de l'invention apparaîtront à l'examen de la description détaillée d'un mode de réalisation de l'invention nullement limitatif, et des dessins annexés, sur lesquels :
- la figure 1 représente une vue schématique d'un dispositif selon l'invention et d'une unité de pyrogazéification,
- la figure 2 représente une vue schématique plus détaillée d'un dispositif selon l'invention comprenant une sous-unité de refroidissement et de lavage, une sous-unité de méthanisation et une sous-unité de biométhanation ainsi qu'une unité de pyrogazéification.

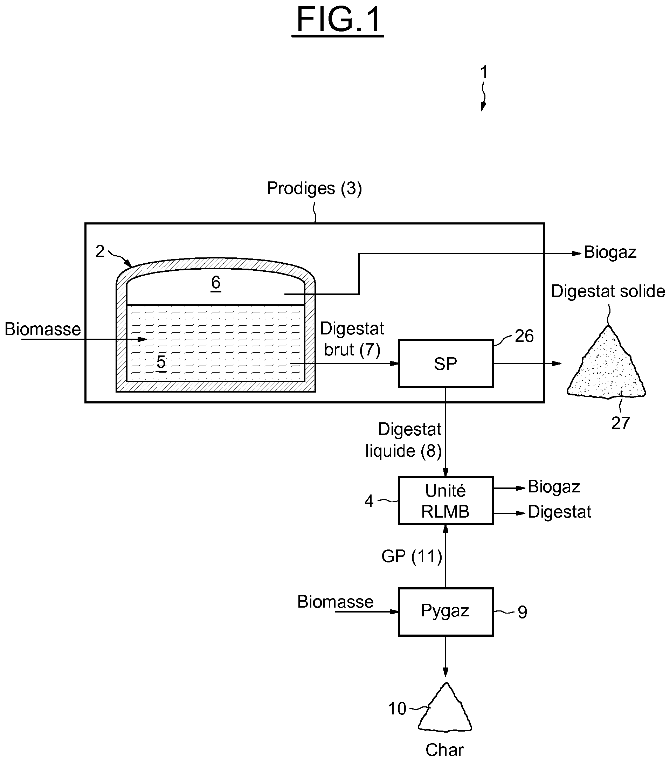

Sur la figure 1 est représenté de manière schématique un dispositif 1, destiné à transformer du gaz de pyrogazéification GP 11 en biogaz, comprenant un digesteur anaérobie 2 équipée d'un équipement de séparation de phases 26, formant ensemble une unité de production de digestat liquide Prodiges 3, nommée Prodiges 3, et une unité fonctionnelle 4, dénommé unité RLMB 4, comprenant une fonction de refroidissement R, une fonction de lavage L, une fonction de méthanisation M et une fonction de biométhanation B.

Le digesteur anaérobie 2 comprend une cuve remplie de digestat 5 surmontée d'une membrane étanche constituant un espace de stockage, lequel est rempli de biogaz 6. Sur la figure 1, la cuve présente une forme essentiellement cylindrique mais peut aussi présenter une forme cubique ou parallélépipédique.

La cuve du digesteur 2 peut être fabriquée à partir d'acier, de béton ou de tout autre matériau tandis que la membrane étanche peut être réalisée en polymère, notamment en EPDM (éthylène/propylène/diène monomère).

Le digesteur anaérobie 2 est alimenté en biomasse, laquelle biomasse est dégradée par des bactéries, par la mise en œuvre d'une réaction biologique de méthanisation (encore appelée digestion anaérobie), dans des conditions contrôlées et ceci en absence d'oxygène. Les productions du digesteur anaérobie 2 sont, d'une part du digestat brut 7, et d'autre part du biogaz 6 essentiellement constitué de méthane et de monoxyde de carbone.

Le digestat brut 7 possède une siccité généralement comprise entre 4 et 12%. L'unité de production de digestat Prodiges 3 comprend également en aval du digesteur anaérobie 2 un système de séparation de phase 26, dénommé SP 26, destiné à séparer le digestat brut 7 en une fraction liquide, appelée digestat liquide 8, et une fraction solide, appelée digestat solide 27.

Le digestat liquide 8 possède une siccité, généralement comprise entre 3 et 10%, tandis que le digestat solide 27 possède une siccité généralement comprise entre 20 et 30%.

Le digestat liquide 8 contient des matières solides en faible concentration et surtout de faible granulométrie, et peut donc circuler aisément à travers les différentes sous-unités de l'unité fonctionnelle RLMB 4 car il est plus facile à pomper dans les circuits d'acheminement et à agiter dans les différentes cuves de l'unité fonctionnelle RLMB 4.

L'unité de production de digestat liquide Prodiges 3, équipé du système SP 26, constitue donc une unité capable de produire du biogaz 6 et du digestat liquide 8 contenant des bactéries anaérobies.

La totalité ou une partie du digestat liquide 8 produit est transférée vers l'unité fonctionnelle RLMB 4 afin d'alimenter chacune des fonctions de méthanisation M et de biométhanation B, et de préférence chacune des fonctions de refroidissement R et de lavage L.

La biomasse alimentant le digesteur 2 peut être solide ou liquide. Elle peut correspondre à des matières organiques d'origine végétale ou animale et être issues d'activités agricoles (pailles, fumiers...), industrielles (déchets d'abattoirs, déchet des préparations alimentaires,...) ou encore urbaines (déchets de restauration collective, de la grande distribution, collecte sélective organique,...). Les biomasses solides ont une siccité généralement comprise entre 15 et 30%, mais peut dans certains cas aller jusqu'à des valeurs proches de 100% (par exemple des pailles). Les biomasses liquides ont une siccité moyenne généralement comprise entre 3 et 15%.

De préférence, les biomasses alimentant le digesteur 2 sont constituées de biomasse saine, relativement humide, ne contenant pas de produits toxiques ou indésirables et à dégradation lente, de telle sorte que les bactéries qui se développeront dans le digestat 5 se trouvent dans un environnement favorable afin de favoriser leur nombre, leur résistance, leur robustesse, leur efficacité et leur variété.

En effet, même si l'unité de production de digestat liquide Prodiges 3 produit du biogaz 6, et du digestat solide 27, sa fonction première est de produire de façon abondante un digestat liquide 8 de bonne qualité, c'est-à-dire constitué de bactéries résistantes, robustes, en grand nombre, et appartenant à une large variété de familles.

La figure 1 représente également une unité de pyrogazéification 9, dénommée unité Pygaz 9, qui est alimentée en matières susceptibles d'être traitées dans une unité de pyrogazéification, qui peuvent être de nature très diverses.

Elles correspondent en général à des matières d'origine organique non fossiles, transformées ou non, ou bien à des matières organiques dérivées du pétrole. Parmi les grandes familles de matières d'origine organique non fossiles opportunément valorisables par pyrogazéification, on peut citer principalement, et de manière non exhaustive, les biomasses végétales d'origine agricole (le bois, les pailles, les menu-pailles et autres résidus de culture, les cultures énergétiques etc.), les déchets industriels banals non inertes et les déchets issus d'une collecte sélective de matières organiques d'origine municipale, (déchets de bois, papiers, cartons, textiles, etc.) ou encore les déchets d'activités de d'épuration (boues de stations d'épuration, etc.). Les matières dérivées du pétrole valorisables sont principalement des déchets d'objets en matière plastiques (résidus urbains d'objets en plastique de la vie courante, sac et autres emballages plastique, pneus, résidus de décharges automobiles etc.).

A la différence des biomasses qui alimentent l'unité de production de digestat liquide Prodiges 3, qui sont le plus souvent très humides, les matières susceptibles d'être traitées dans une unité de pyrogazéification sont de préférence les plus sèches possibles.

Une réaction de pyrogazéification se déroule en présence d'une quantité réduite ou en l'absence d'oxygène à des températures élevées, pouvant aller de 300 à 1200°C au sein de l'unité Pygaz 9, afin de transformer la biomasse, d'une part, en un mélange de gaz incondensables et de matières condensables, appelé gaz de pyrogazéification, et, d'autre part, en charbon aussi appelé char 10.

Le gaz de pyrogazéification, produit par l'unité Pygaz 9, est transféré vers l'unité fonctionnelle RLMB 4.

Le gaz de pyrogazéification est d'abord refroidi lors de l'accomplissement de la fonction de refroidissement R de l'unité RLMB 4.

La fonction de refroidissement est de préférence réalisée par injection du gaz de pyrogazéification dans un liquide froid ou refroidi, dit liquide de refroidissement et/ou par aspersion du gaz de pyrogazéification par un tel liquide de refroidissement.

Le liquide de refroidissement est de préférence constitué en totalité ou en partie par du digestat liquide 8 produit par l'unité de production de digestat liquide Prodiges 3.

Le gaz de pyrogazéification est ensuite lavé lors de l'accomplissement de la fonction de lavage L de l'unité RLMB 4.

La fonction de lavage est de préférence réalisée par injection du gaz de pyrogazéification dans un liquide froid ou refroidi, dit liquide de lavage, et/ou par aspersion du gaz de pyrogazéification par un tel liquide de lavage. Ledit liquide de lavage a notamment de préférence une température comprise entre 35 et 50°C.

Le liquide de lavage est de préférence constitué en totalité ou en partie par du digestat liquide 8 produit par l'unité de production de digestat liquide Prodiges 3.

La fonction de lavage L de l'unité RLMB 4 sépare, d'une part, les sous-produits condensables du gaz de pyrogazéification ainsi que les particules fines solides et, d'autre part, le gaz de synthèse, ou syngaz, composé essentiellement de gaz incondensables, principalement du monoxyde de carbone, du dihydrogène, communément appelé hydrogène, du méthane et du dioxyde de carbone.

Les gaz condensables et les matières solides, nommés les sous-produits, contenus dans le gaz de pyrogazéification, sont séparés du syngaz par la fonction de lavage L pour être ensuite acheminés vers la fonction de méthanisation M de l'unité RLMB 4. De préférence, ces sous-produits du gaz de pyrogazéification sont d'abord mélangés dans la fonction de lavage avec du digestat liquide 8, produit par l'unité de production de digestat liquide Prodiges 3, avant d'être transférés vers la fonction de méthanisation M de l'unité RLMB 4.

La fonction de méthanisation M met en œuvre une réaction de méthanisation biologique des sous-produits issus du gaz de pyrogazéification pour produire du biogaz.

Ainsi la fonction de méthanisation M valorise les sous-produits contenus dans le gaz de pyrogazéification en les transformant en biogaz.

La fonction de biométhanation B met en œuvre une réaction de méthanation biologique du syngaz, issu de la fonction de lavage, afin de transformer ledit syngaz en biogaz.

Les fonctions de méthanisation M et de biométhanation B de l'unité RLMB 4 produisent du biogaz respectivement à partir des sous-produits contenus dans le gaz de pyrogazéification, et du syngaz, les deux produits étant séparés et produits dans la fonction de lavage L.

Les fonctions de méthanisation M et de biométhanation B de l'unité RLMB 4 produisent également du digestat excédentaire, qui n'est plus biodégradable suite aux réactions de méthanisation et de biométhanation. Le digestat excédentaire peut généralement servir avantageusement pour l'épandage agricole.

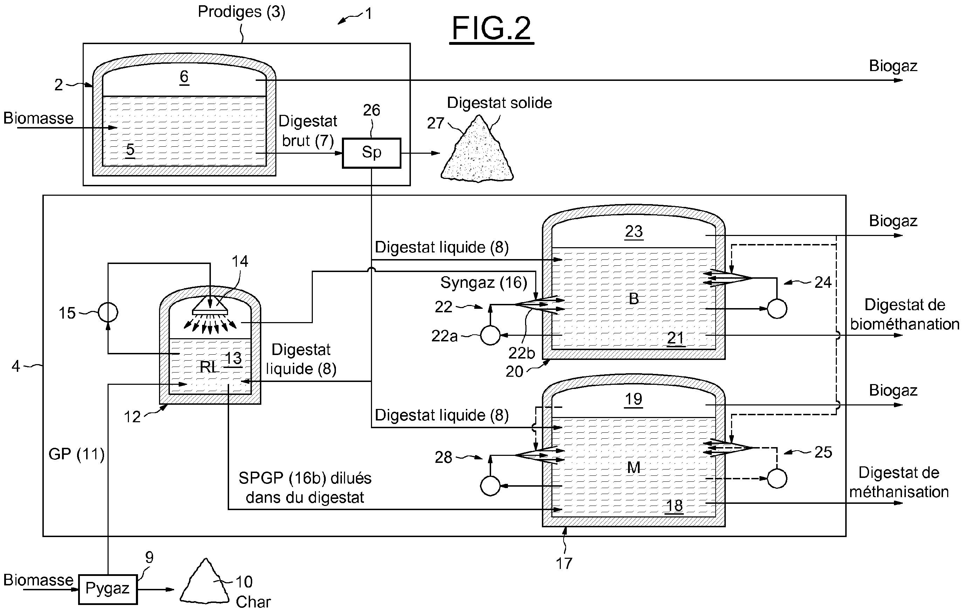

La figure 2 représente le dispositif 1 de manière plus détaillée selon un mode de réalisation de l'unité RLMB 4, ainsi qu'une série de dispositifs permettant de traiter le digestat liquide 8 produit par l'unité de production de digestat liquide Prodiges 3.

L'unité RLMB 4 comprend une sous-unité 12 de refroidissement et de lavage, dénommé sous-unité RL 12, une sous-unité 17 de méthanisation, dénommé sous-unité M 17, et une sous-unité 20 de biométhanation, dénommé sous-unité B 20.

La sous-unité RL 12 est un dispositif dans lequel le gaz de pyrogazéification 11, dénommé GP 11, circule dans un liquide 13 froid ou refroidi constituant un liquide de refroidissement et de lavage ayant, de préférence, une température comprise entre 35 et 50°C.

Le liquide 13 de refroidissement et de lavage permet à la fois de refroidir et laver le gaz de pyrogazéification GP 11 produit par l'unité Pygaz 9.

Dans un premier temps, le liquide 13 permet d'abaisser la température du gaz de pyrogazéification GP 11 qui présente, en sortie de l'unité Pygaz 9, une température élevée pouvant aller de 300 à 1.200°C. Cet abaissement de la température facilite le traitement ultérieur du gaz de pyrogazéification GP 11.

Dans un second temps, le liquide 13 permet de laver le gaz de pyrogazéification GP 11, c'est-à-dire de séparer les sous-produits, dénommés SPGP 16b du gaz de pyrogazéification et le syngaz 16, qui est un mélange de gaz incondensables. Les sous-produits SPGP 16b du gaz de pyrogazéification 11 sont constitués par des goudrons, des huiles, d'autres gaz condensables ainsi que des particules fines solides, dont des cendres, des particules de charbon et des particules minérales. Les sous-produits SPGP 16b refroidis comportent des gaz condensables sous forme liquide et des matières solides. En sortie de l'unité Pygaz 9, ces sous-produits SPGP 16b sont présents dans le gaz de pyrogazéification GP 11 sous forme gazeuse ou sous forme de particules fines, en mélange avec le syngaz 16.

Ainsi le liquide 13 favorise la condensation des goudrons, des huiles et des autres matières condensables sous forme de liquide. Ces sous-produits liquides captent, au cours de leur condensation, les particules fines solides, soit donc la partie solide des sous-produits SPGP 16b.

La séparation des sous-produits SPGP 16b dans le gaz de pyrogazéification GP 11 conduit à la formation d'un mélange de gaz incondensables à température ambiante constitué essentiellement de monoxyde de carbone, d'hydrogène, de méthane et de dioxyde de carbone. Le mélange de gaz, incondensables à température ambiante, ainsi obtenu correspond au gaz de synthèse ou syngaz 16.

Le syngaz 16 présente donc un degré de pureté plus élevé que le gaz de pyrogazéification GP 11. Il est donc plus aisément utilisable et valorisable, car les sous-produits SPGP 16b, responsables notamment du caractère initialement encrassant et corrosif du gaz de pyrogazéification GP 11, ont été séparés dans la sous-unité RL 12.

La sous-unité RL 12 est équipée en complément d'une douche 14 qui a pour fonction l'aspersion intense, à l'aide du liquide de refroidissement et de lavage 13, le gaz de pyrogazéification GP 11, afin de favoriser son refroidissement et son lavage. Pour ce faire, une partie du liquide 13 est pompée au moyen d'une pompe de circulation 15 qui achemine ce liquide 13 à la douche 14. La présence de la douche 14 est conforme à de nombreux procédés classiques mis en œuvre dans les technologies des tours de lavage de gaz.

Le liquide de refroidissement et de lavage 13 peut être constitué en totalité ou en partie par du digestat liquide 8 produit par l'unité de production de digestat liquide Prodiges 3. De préférence, le liquide 13 est constitué en totalité par le digestat liquide 8.

Les sous-produits SPGP 16b, en solution dans le liquide 13, sont ensuite acheminés vers la sous-unité de méthanisation M 17.

La sous-unité M 17 est constituée de préférence par un digesteur anaérobie comprenant une cuve surmontée d'une membrane étanche constituant un espace de stockage du biogaz.

Dans la sous-unité M 17, les sous-produits SPGP 16b, en mélange avec le liquide de refroidissement et de lavage 13, sont dégradés par des bactéries anaérobies présentes dans le digestat 18, afin d'être transformés en biogaz 19. Par ce biais, les sous-produits SPGP 16b sont ainsi valorisés en biogaz 19 grâce à la mise en œuvre d'une réaction biologique de méthanisation.

Le digestat liquide 18 peut provenir en totalité ou en partie du transfert de digestat liquide 8 produit par l'unité de production de digestat liquide Prodiges 3. De préférence, le digestat liquide 18 provient en totalité du transfert du digestat liquide 8 produit par l'unité de production de digestat liquide Prodiges 3.

Le digestat excédentaire de l'unité de méthanisation M 17, est ensuite évacué.

Le syngaz 16 issu de l'unité RL 12 est acheminé vers la sous-unité B 20.

La sous-unité B 20 est de préférence un digesteur anaérobie comprenant une cuve surmontée d'une membrane étanche constituant un espace de stockage de biogaz.

Dans la sous-unité B 20, le syngaz 16 est dissout dans le digestat 21, puis il est transformé en biogaz 23 par l'action des bactéries présentes dans le digestat 21. Par ce biais, le syngaz 16 est transformé par voie biologique en biogaz 23. En particulier, le mélange de monoxyde de carbone, de dioxyde de carbone et d'hydrogène présents dans le syngaz 16 est transformé en méthane. L'excès probable de dioxyde de carbone dans ce mélange conduit donc finalement à la production d'un mélange de méthane et de dioxyde de carbone, qui constitue essentiellement le biogaz 23.

Le digestat liquide 21 peut provenir, en totalité ou en partie, du transfert du digestat liquide 8 produit par l'unité de production de digestat liquide Prodiges 3. De préférence, le digestat liquide 21 provient en totalité du transfert du digestat liquide 8 produit par l'unité de production de digestat liquide Prodiges 3.

Dans certains cas, la réaction de biométhanation du syngaz 16 dans la sous-unité B 20 peut être incomplète, notamment lorsque la totalité du monoxyde carbone et de l'hydrogène n'est pas transformée en méthane.

En effet, la biométhanisation du syngaz 16 peut être limitée par la difficulté de la dissolution d'une grande quantité de monoxyde de carbone, de dioxyde de carbone et/ou d'hydrogène dans un volume de digestat liquide 21 limité. En effet, les bactéries responsables de la biométhanation peuvent être efficientes mais leur action peut être limitée par la capacité trop faible de l'installation à transférer une masse suffisante de monoxyde de carbone, de dioxyde de carbone et/ou d'hydrogène dans le milieu liquide qui est celui des bactéries. Ceci peut se produire dans le cas où le volume de digestat présent dans la sous-unité B 20 est trop faible par rapport à la quantité de syngaz à transformer en biogaz. De préférence, le volume de la cuve de la sous-unité B 20 est donc le plus grand possible.

Afin de surmonter cet inconvénient, le syngaz 16 peut être injecté dans la sous-unité B 20 sous forme de microbulles pour favoriser la dissolution du monoxyde de carbone, du dioxyde de carbone et de l'hydrogène dans le digestat liquide 21.

Par exemple, un hydro-éjecteur 22 peut être utilisé afin d'injecter le syngaz 16 sous forme de microbulles dans le digestat liquide 18.

L'hydro-éjecteur 22 peut être composé d'une pompe à pression 22a qui aspire le digestat liquide 21 dans la sous-unité B 20 et le réinjecte dans la sous-unité B 20 à travers un système de venturi 22b, dans l'aspiration duquel est injecté le syngaz 16. Il en résulte une forte turbulence associée à une dépression, ayant pour effet de mélanger intimement le syngaz 16 sous forme de fines bulles avec le digestat liquide 21, ce qui a pour conséquence une dilution plus importante, dans le digestat liquide 21, du monoxyde de carbone, du dioxyde de carbone et de l'hydrogène présents dans le syngaz 16.

La biométhanation du syngaz 16 peut être aussi incomplète dans le cas où la quantité ou la qualité de flore bactérienne disponible dans la sous-unité B 20 est insuffisante pour traiter les quantités de monoxyde de carbone, de dioxyde de carbone et d'hydrogène présentes dans le syngaz 16 à transformer.

Dans ce cas, le syngaz 16 peut être soumis à plusieurs réactions de biométhanation successives.

Ainsi le biogaz 23 est de préférence aspiré et réinjecté dans le digestat liquide 21 dans la partie inférieure de la cuve de la sous-unité B 20, par exemple au moyen d'un hydro-éjecteur 24. De cette manière, les gaz constituant le syngaz 16, qui n'ont pas été transformés en méthane et dioxyde de carbone dans la sous-unité B 20, sont réinjectés dans le digestat liquide 21 plusieurs fois de suite afin d'être soumis plusieurs fois à un processus de biométhanation.

Par ailleurs, dans un mode de réalisation, le biogaz 23 peut être aspiré et injecté dans la sous-unité M 17, en particulier dans le digestat liquide 18 au niveau de la partie inférieure de la cuve de la sous-unité M 17. Ainsi les gaz du syngaz 16, non transformés en méthane et dioxyde de carbone dans la sous-unité B 20, et qui sont encore présents dans le biogaz 23 peuvent être réinjectés dans le digestat 18 de la sous-unité M 17 où ils peuvent être soumis à un nouveau processus de biométhanation. Dans ce cas, la fonction de biométhanation est mise en œuvre principalement dans la sous-unité B 20 et est complétée dans la sous-unité M 17. En d'autres termes, la sous-unité M 17 peut compléter la réaction de biométhanation de la sous-unité B 20 en transformant en biogaz les gaz du syngaz 16 n'ayant pas complètement été transformés en biogaz dans la sous-unité M 17.

De manière complémentaire, le biogaz 23, produit dans la sous-unité M 17, peut être aspiré et réinjecté dans le digestat liquide 18 au niveau de la partie inférieure de la cuve de la sous-unité M 17, par exemple au moyen d'un hydro-éjecteur 28. De cette manière, les gaz constituant le syngaz 16, qui n'ont pas été transformés en méthane et en dioxyde de carbone dans la sous-unité B 20 et lors de leur passage complémentaire dans la sous-unité M 17, qui sont encore présents dans le biogaz, sont de préférence réinjectés plusieurs fois dans le digestat de la sous-unité M 17.

Il en résulte que la réaction de biométhanation se déroulant dans la sous-unité B 20 peut être complétée dans la sous-unité M 17 à une ou plusieurs reprises.

L'unité de production de digestat liquide Prodiges 3 a pour fonction de produire du digestat liquide 8 afin d'alimenter l'unité RLMB 4. En particulier, la figure 2 illustre que l'unité de production de digestat liquide Prodiges 3 alimente la sous-unité RL 12, la sous-unité M 17 et la sous-unité B 20.

Plus particulièrement, l'unité de production de digestat liquide Prodiges 3 alimente en digestat liquide 8 les différentes sous-unités de l'unité RLMB 4 de manière régulière, de façon séquentielle ou de préférence continue, dans le but, notamment, de diminuer les concentrations en SPGP 16b dans la sous-unité M 17.

En effet, l'alimentation en quantité importante de digestat liquide dans la sous-unité M 17 a pour effet de diluer les digestats déjà présents dans ladite sous-unité. Cela permet, en premier lieu, de diminuer les concentrations dans la sous-unité M 17 des substances agressives qui proviennent des gaz de pyrogazéification, et sont potentiellement toxiques ou inhibitrices à certaines concentrations, vis-à-vis de la flore bactérienne. Une telle diminution des concentrations des diverses substances agressives, potentiellement toxiques ou inhibitrices, vis-à-vis de la flore bactérienne, est susceptible de favoriser l'activité, et donc le rendement, des bactéries à l'œuvre dans la fonction méthanisation de l'unité fonctionnelle RLMB 4.

De plus, l'unité de production de digestat liquide Prodiges 3 alimente en digestat liquide 8 les sous-unités de méthanisation et de biométhanation de l'unité RLMB 4 de manière régulière, de façon séquentielle ou de préférence continue, dans le but d'entretenir la diversité des familles des bactéries et de renforcer ou de remplacer les bactéries qui sont affaiblies ou qui disparaissent au cours de la réaction de méthanisation ou de biométhanation, à l'oeuvre respectivement dans la sous-unité M 17 et la sous-unité B 20. Ainsi cette alimentation en digestat liquide permet d'augmenter la quantité, la variété, la qualité, la résistance et l'efficacité des bactéries responsables de la méthanisation des sous-produits du gaz de pyro gazéification.

L'augmentation de la production de digestat liquide 8 est réalisée par une augmentation de la quantité de biomasse à traiter, en particulier de biomasse humide, dans l'unité de production de digestat liquide Prodiges 3.

L'unité de production de digestat liquide Prodiges 3 produit un digestat liquide 8 ayant une siccité généralement comprise entre 3 et 10%.

D'autres modes de réalisation peuvent être mis en œuvre dans lesquels l'unité RLMB 4 comprend un quelconque agencement d'une ou plusieurs sous-unités distinctes susceptibles de réaliser chacune tout ou partie de la ou des fonctions de refroidissement, de lavage, de méthanisation et/ou de biométhanation.

Ainsi, selon divers modes de réalisation, l'unité RLMB 4 peut être constituée de quatre, ou de trois, d'une seule ou de deux sous-unités distinctes susceptibles de réaliser ensembles les quatre fonctions de refroidissement, de lavage, de méthanisation et de biométhanation.

Par ailleurs, dans la figure 2, la sous-unité RL 12, la sous-unité M17 et la sous-unité B20 ont été respectivement représentées comme étant constituées d'une seule cuve.

Cependant, d'autres modes de réalisation peuvent être mis en œuvre dans lesquels chacune des sous-unités précédemment décrites peut comprendre une ou plusieurs cuves.

## Revendications

1. Dispositif (1) de transformation par voie biologique de gaz de pyrogazéification (11) en biogaz (19, 23) comprenant :
- une unité de production de digestat liquide (3), comprenant une unité de production de digestat brut (2) et un système de séparation de phase (26), ayant ensemble pour fonction la production de digestat liquide (8) contenant des bactéries anaérobies,
- une unité fonctionnelle RLMB (4) comprenant quatre fonctions :
(i) une fonction de refroidissement R du gaz de pyrogazéification (11),
(ii) une fonction de lavage L du gaz de pyrogazéification (11),
(iii) une fonction de méthanisation M d'au moins une partie des sous-produits du gaz de pyrogazéification SPGP (16b), extraits du gaz de pyrogazéification (11) par les fonctions de refroidissement R et de lavage L, afin de former du biogaz (19),
(iv) une fonction de biométhanation B du syngaz (16), extrait du gaz de pyrogazéification (11) et séparé des sous-produits du gaz de pyrogazéification SPGP (16b) par les fonctions de refroidissement R et de lavage L, afin de former du biogaz (23),
l'unité fonctionnelle RLMB (4) étant alimentée par au moins le gaz de pyrogazéification (11), et par du digestat provenant, pour tout ou partie, de l'unité de production de digestat liquide (3),
l'unité fonctionnelle RLMB (4) comprenant une ou plusieurs sous-unités distinctes susceptibles de réaliser chacune tout ou partie d'une ou plusieurs des fonctions de refroidissement, de lavage, de méthanisation et/ou de biométhanation.

2. Dispositif (1) selon la revendication 1, **caractérisé en ce que** la fonction de méthanisation est alimentée au moins par du digestat provenant, pour tout ou partie, de l'unité de production de digestat liquide (3).

3. Dispositif (1) selon la revendication 1 ou 2, **caractérisé en ce que** la fonction de méthanation est alimentée au moins par du digestat provenant en totalité de l'unité de production de digestat liquide (3).

4. Dispositif (1) selon l'une quelconque des revendications précédentes, **caractérisé en ce que** la fonction de méthanisation est accomplie par une sous-unité de méthanisation (17) constituée d'un ou plusieurs digesteurs anaérobies.

5. Dispositif (1) selon l'une quelconque des revendications précédentes, **caractérisé en ce que** la fonction de biométhanation est accomplie par une sous-unité de biométhanation (20) constituée d'un ou plusieurs digesteurs anaérobies.

6. Dispositif (1) selon l'une quelconque des revendications précédentes, **caractérisé en ce que** le digestat alimentant l'unité fonctionnelle RLMB (4) est constitué en totalité par du digestat liquide (8) produit par l'unité de production de digestat liquide (3).

7. Dispositif (1) selon l'une quelconque des revendications précédentes, **caractérisé en ce que** l'unité fonctionnelle RLMB (4) comprend une sous-unité RL (12) susceptible de réaliser à la fois la fonction de refroidissement R et la fonction de lavage L du gaz de pyrogazéification (11).

8. Dispositif (1) selon l'une quelconque des revendications 1 à 6, **caractérisé en ce que** l'unité fonctionnelle RLMB (4) comprend une sous-unité susceptible de réaliser à la fois la fonction de lavage L et la fonction de méthanisation M.

9. Procédé de transformation par voie biologique du gaz de pyrogazéification (11) en biogaz (19, 23) comprenant :
- une étape d'alimentation d'une unité fonctionnelle RLMB (4), telle que définie selon l'une quelconque des revendications 1 à 8, par du digestat contenant des bactéries anaérobies, lequel digestat est constitué au moins en partie par le digestat liquide (8) produit par une unité de production de digestat liquide (3) telle que définie selon la revendication 1,
- une étape de transfert du gaz de pyrogazéification (11) vers l'unité fonctionnelle RLMB (4), puis
- une étape de refroidissement du gaz de pyrogazéification (11),
- une étape de lavage du gaz de pyrogazéification (11) pour séparer, d'une part, le syngaz (16) et, d'autre part, les sous-produits du gaz de pyrogazéification SPGP (16b),
- une étape de méthanisation par voie biologique d'au moins une partie des sous-produits du gaz de pyrogazéification SPGP (16b), extraits du gaz de pyrogazéification (11) lors des étapes de refroidissement et de lavage, pour produire au moins du biogaz (19),
- une étape de méthanation par voie biologique, ou biométhanation, du syngaz (16), extrait du gaz de pyrogazéification (11) lors des étapes de refroidissement et de lavage, pour produire au moins du biogaz (23).

10. Procédé selon la revendication 9, **caractérisé en ce que** l'étape de refroidissement est mise en œuvre par injection du gaz de pyrogazéification (11) dans un liquide de refroidissement, et/ou par aspersion du gaz de pyrogazéification (11) par un liquide de refroidissement, lequel liquide de refroidissement est constitué pour tout ou partie par le transfert du digestat liquide (8).

11. Procédé selon la revendication 9 ou 10, **caractérisé en ce que** l'étape de lavage est mise en œuvre par injection du gaz de pyrogazéification (11) dans un liquide de lavage, et/ou par aspersion du gaz de pyrogazéification (11) par un liquide de lavage, lequel liquide de lavage est constitué pour tout ou partie par le transfert du digestat liquide (8).

12. Procédé selon la revendication 9, **caractérisé en ce que** l'étape de refroidissement est mise en œuvre par échange de chaleur au moyen d'un échangeur de chaleur sans injection et/ou aspersion du gaz de pyrogazéification (11) dans un liquide.

13. Procédé selon l'une quelconque des revendications 9 à 12, **caractérisé en ce qu'**il comprend en outre une étape de séparation d'une partie des sous-produits du gaz de pyrogazéification SPGP (16b) au cours de l'étape de refroidissement et/ou de lavage avant l'étape de méthanisation.

## Patentansprüche

1. Vorrichtung (1) zur biologischen Umwandlung von Pyrovergasungsgas (11) in Biogas (19, 23), umfassend:
- eine Einheit zur Produktion von flüssigem Gärrest (3), umfassend eine Einheit zur Produktion von rohem Gärrest (2) und ein Phasentrennungssystem (26), die gemeinsam die Funktion der Produktion von flüssigem Gärrest (8), der anaerobe Bakterien enthält, haben,
- eine KSMB-Funktionseinheit (4), die vier Funktionen umfasst:
(i) eine Kühlungsfunktion K des Pyrovergasungsgases (11),
(ii) eine Spülungsfunktion S des Pyrovergasungsgases (11),
(iii) eine Methanisierungsfunktion M von mindestens einem Teil von Nebenprodukten des Pyrovergasungsgases NPPG (16b), die durch die Kühlungs- K und die Spülungsfunktion S aus dem Pyrovergasungsgas (11) extrahiert werden, um Biogas (19) zu bilden,
(iv) eine Biomethanierungsfunktion B von Syngas (16), das durch die Kühlungs- K und die Spülungsfunktion S aus dem Pyrovergasungsgas (11) extrahiert wird und von Nebenprodukten des Pyrovergasungsgases NPPG (16b) getrennt wird, um Biogas (23) zu bilden,
wobei die KSMB-Funktionseinheit (4) mit mindestens dem Pyrovergasungsgas (11) und mit dem Gärrest gespeist wird, der vollständig oder zum Teil von der Einheit (3) zur Produktion von flüssigem Gärrest stammt,
wobei die KSMB-Funktionseinheit (4) eine oder mehrere verschiedene Untereinheiten umfasst, die dazu geeignet sind, jeweils eine oder mehrere der Kühlungs-, der Spülungs-, der Methanisierungs- und/oder der Biomethanierungsfunktion vollständig oder zum Teil durchzuführen.

2. Vorrichtung (1) nach Anspruch 1, **dadurch gekennzeichnet, dass** die Methanisierungsfunktion mindestens mit dem Gärrest gespeist wird, der vollständig oder zum Teil von der Einheit zur Produktion von flüssigem Gärrest (3) stammt.

3. Vorrichtung (1) nach Anspruch 1 oder 2, **dadurch gekennzeichnet, dass** die Methanierungsfunktion mindestens mit dem Gärrest gespeist wird, der komplett von der Einheit zur Produktion von flüssigem Gärrest (3) stammt.

4. Vorrichtung (1) nach einem der vorhergehenden Ansprüche, **dadurch gekennzeichnet, dass** die Methanisierungsfunktion von einer Methanisierungsuntereinheit (17) ausgeführt wird, die aus einem oder mehreren anaeroben Faulbehältern besteht.

5. Vorrichtung (1) nach einem der vorhergehenden Ansprüche, **dadurch gekennzeichnet, dass** die Biomethanierungsfunktion von einer Biomethanierungsuntereinheit (20) ausgeführt wird, die aus einem oder mehreren anaeroben Faulbehältern besteht.

6. Vorrichtung (1) nach einem der vorhergehenden Ansprüche, **dadurch gekennzeichnet, dass** der Gärrest, der die KSMB-Funktionseinheit (4) speist, komplett aus flüssigem Gärrest (8) besteht, der von der Einheit (3) zur Produktion von flüssigem Gärrest produziert wird.

7. Vorrichtung (1) nach einem der vorhergehenden Ansprüche, **dadurch gekennzeichnet, dass** die KSMB-Funktionseinheit (4) eine KS-Untereinheit (12) umfasst, die dazu geeignet ist, gleichzeitig die Kühlungsfunktion K und die Spülungsfunktion S für das Pyrovergasungsgas (11) durchzuführen.

8. Vorrichtung (1) nach einem der Ansprüche 1 bis 6, **dadurch gekennzeichnet, dass** die KSMB-Funktionseinheit (4) eine Untereinheit umfasst, die dazu geeignet ist, gleichzeitig die Spülungsfunktion S und die Methanisierungsfunktion M durchzuführen.

9. Verfahren zur biologischen Umwandlung von Pyrovergasungsgas (11) in Biogas (19, 23), umfassend:
- einen Schritt einer Speisung einer KSMB-Funktionseinheit (4), wie in einem der Ansprüche 1 bis 8 definiert, mit Gärrest, der anaerobe Bakterien enthält, wobei der Gärrest mindestens zum Teil aus dem flüssigen Gärrest (8) besteht, der von einer Einheit zur Produktion von flüssigem Gärrest (3), wie in Anspruch 1 definiert, produziert wird,
- einen Schritt einer Überführung des Pyrovergasungsgases (11) zu der KSMB-Funktionseinheit (4), dann
- einen Schritt einer Kühlung des Pyrovergasungsgases (11),
- einen Schritt einer Spülung des Pyrovergasungsgases (11), um einerseits Syngas (16) und andererseits Nebenprodukte des Pyrovergasungsgases NPPG (16b) zu trennen,
- einen Schritt einer biologischen Methanisierung von mindestens einem Teil von Nebenprodukten des Pyrovergasungsgases NPPG (16b), die während den Schritten der Kühlung und der Spülung aus dem Pyrovergasungsgas (11) extrahiert werden, um mindestens Biogas (19) zu produzieren,
- einen Schritt einer biologischen Methanierung oder Biomethanierung des Syngases (16), das während den Schritten der Kühlung und der Spülung aus dem Pyrovergasungsgas (11) extrahiert wird, um mindestens Biogas (23) zu produzieren.

10. Verfahren nach Anspruch 9, **dadurch gekennzeichnet, dass** der Schritt der Kühlung durch Injektion des Pyrovergasungsgases (11) in eine Kühlflüssigkeit und/oder durch Besprengung des Pyrovergasungsgases (11) mit einer Kühlflüssigkeit umgesetzt wird, wobei die Kühlflüssigkeit vollständig oder zum Teil durch die Überführung des flüssigen Gärrests (8) gebildet wird.

11. Verfahren nach Anspruch 9 oder 10, **dadurch gekennzeichnet, dass** der Schritt der Spülung durch Injektion des Pyrovergasungsgases (11) in eine Spülflüssigkeit und/oder durch Besprengung des Pyrovergasungsgases (11) mit einer Spülflüssigkeit umgesetzt wird, wobei die Spülflüssigkeit vollständig oder zum Teil durch die Überführung des flüssigen Gärrests (8) gebildet wird.

12. Verfahren nach Anspruch 9, **dadurch gekennzeichnet, dass** der Schritt der Kühlung durch Wärmeaustausch mittels eines Wärmeaustauschers ohne Injektion und/oder Besprengung des Pyrovergasungsgases (11) in eine Flüssigkeit umgesetzt wird.

13. Verfahren nach einem der Ansprüche 9 bis 12, **dadurch gekennzeichnet, dass** es weiterhin einen Schritt einer Trennung eines Teils von Nebenprodukten des Pyrovergasungsgases NPPG (16b) im Verlauf des Schrittes der Kühlung und/oder der Spülung vor dem Schritt der Methanisierung umfasst.

## Claims

1. Device (1) for biologically transforming pyrogasification gas (11) into biogas (19, 23) comprising:
- a liquid digestate production unit (3), comprising a raw digestate production unit (2) and a phase separation system (26), with the joint function of producing liquid digestate (8) containing anaerobic bacteria,
- an RLMB functional unit (4) comprising four functions:
(i) a cooling function R of the pyrogasification gas (11),
(ii) a washing function L of the pyrogasification gas (11),
(iii) a methanisation function M of at least a portion of the by-products of the pyrogasification gas SPGP (16b), extracted from the pyrogasification gas (11) by the cooling function R and washing function L, so as to form biogas (19),
(iv) a biomethanation function B of the syngas (16), extracted from the pyrogasification gas (11) and separated from the by-products of the pyrogasification gas SPGP (16b) by the cooling function R and washing function L, so as to form biogas (23),
the RLMB functional unit (4) being supplied by at least the pyrogasification gas (11), and by digestate originating wholly or partly from the liquid digestate production unit (3),
the RLMB functional unit (4) comprising one or more separate sub-units each capable of performing all or some of one or more of the cooling, washing, methanisation and/or biomethanation functions.

2. Device (1) according to claim 1, **characterised in that** the methanisation function is supplied at least by digestate originating wholly or partly from the liquid digestate production unit (3).

3. Device (1) according to claim 1 or 2, **characterised in that** the methanation function is supplied at least with digestate originating wholly from the liquid digestate production unit (3).

4. Device (1) according to any of the preceding claims, **characterised in that** the methanisation function is performed by a methanisation sub-unit (17) formed by one or more anaerobic digesters.

5. Device (1) according to any of the preceding claims, **characterised in that** the biomethanation function is performed by a biomethanation sub-unit (20) formed by one or more anaerobic digesters.

6. Device (1) according to any of the preceding claims, **characterised in that** the digestate supplying the RLMB functional unit (4) consists entirely of liquid digestate (8) produced by the liquid digestate production unit (3).

7. Device (1) according to any of the preceding claims, **characterised in that** the RLMB functional unit (4) comprises an RL sub-unit (12) capable of performing both the cooling function R and the washing function L of the pyrogasification gas (11).

8. Device (1) according to any of claims 1 to 6, **characterised in that** the RLMB functional unit (4) comprises a sub-unit which is capable of performing both the washing function L and the methanisation function M.

9. Method for biologically transforming pyrogasification gas (11) into biogas (19, 23) comprising:
- a step of supplying an RLMB functional unit (4), as defined according to any of claims 1 to 8, with digestate containing anaerobic bacteria, which digestate is formed at least partly by the liquid digestate (8) produced by a liquid digestate production unit (3) as defined according to claim 1,
- a step of transferring pyrogasification gas (11) to the RLMB functional unit (4), then
- a step of cooling the pyrogasification gas (11),
- a step of washing the pyrogasification gas (11) for separating on the one hand the syngas (16) and on the other hand the by-products of the pyrogasification gas SPGP (16b),
- a biological methanisation step of at least a portion of the by-products of the pyrogasification gas SPGP (16b), extracted from the pyrogasification gas (11) during the cooling and washing steps to produce at least biogas (19),
- a biological methanation or biomethanation step of the syngas (16), extracted from the pyrogasification gas (11) during the cooling and washing steps to produce at least biogas (23).

10. Method according to claim 9, **characterised in that** cooling step is carried out by injecting pyrogasification gas (11) into a cooling liquid, and/or by spraying pyrogasification gas (11) with a cooling liquid, which cooling liquid consists wholly or partly of the liquid digestate transfer (8).

11. Method according to claim 9 or 10, **characterised in that** the washing step is carried out by injecting pyrogasification gas (11) into a washing liquid, and/or by spraying the pyrogasification gas (11) with a washing liquid, which washing liquid consists wholly or partly of the liquid digestate transfer (8).

12. Method according to claim 9, **characterised in that** the cooling step is carried out by heat exchange by means of a heat exchanger without injection and/or spraying of the pyrogasification gas (11) in a liquid.

13. Method according to any of claims 9 to 12, **characterised in that** it further comprises a step of separating a portion of the by-products from the pyrogasification gas SPGP (16b) during the cooling step and/or the washing step before the methanisation step.
